# EUROPEAN PATENT APPLICATION

(11) **EP 4 495 112 A1**
(43) Date of publication of application: **22.01.2025**
(21) Application number: 24775109.2
(22) Date of filing: 08.03.2024
(51) Int. Cl.: C07D 403/10, C07D 409/14, C07D 405/14, H10K 85/60, H10K 50/11

(54) **NOVEL COMPOUND AND ORGANIC LIGHT-EMITTING DEVICE COMPRISING SAME**

(30) Priority: 17.03.2023 KR 20230035467; 06.03.2024 KR 20240032010
(71) Applicant: LG Chem, Ltd., Yeongdeungpo-gu Seoul 07336 (KR)
(72) Inventor: HEO, Jeonghoe, Daejeon 34122 (KR); JUNG, Min Woo, Daejeon 34122 (KR); KIM, Hoon Jun, Daejeon 34122 (KR); HAN, Miyeon, Daejeon 34122 (KR); LEE, Jungha, Daejeon 34122 (KR); OH, Joongsuk, Daejeon 34122 (KR); CHO, Hye Min, Daejeon 34122 (KR); LEE, Hojung, Daejeon 34122 (KR)
(74) Representative: Hoffmann Eitle
(86) International application number: PCT/KR2024/003029
(87) International publication number: WO 2024/196061

(57) **Abstract**

The present disclosure relates to a novel compound and an organic light emitting device comprising the same.

## Description

### [TECHNICAL FIELD]

### CROSS-REFERENCE TO RELATED APPLICATION(S)

This application claims priority benefit of Korean Patent Application No. 10-2023-0035467 filed on March 17, 2023 and Korean Patent Application No. 10-2024-0032010 filed on March 6, 2024 in the Korean Intellectual Property Office, the disclosures of which are incorporated herein by reference in their entirety.

The present disclosure relates to a novel compound and an organic light emitting device comprising the same.

### [BACKGROUND ART]

In general, an organic light emitting phenomenon refers to a phenomenon where electric energy is converted into light energy by using an organic material. The organic light emitting device using the organic light emitting phenomenon has characteristics such as a wide viewing angle, an excellent contrast, a fast response time, an excellent luminance, driving voltage and response speed, and thus many studies have proceeded.

The organic light emitting device generally has a structure which comprises an anode, a cathode, and an organic material layer interposed between the anode and the cathode. The organic material layer frequently has a multilayered structure that comprises different materials in order to enhance efficiency and stability of the organic light emitting device, and for example, the organic material layer may be formed of a hole injection layer, a hole transport layer, a light emitting layer, an electron transport layer, an electron injection layer, and the like. In the structure of the organic light emitting device, if a voltage is applied between two electrodes, the holes are injected from an anode into the organic material layer and the electrons are injected from the cathode into the organic material layer, and when the injected holes and electrons meet each other, an exciton is formed, and light is emitted when the exciton falls to a ground state again.

There is a continuous need to develop a new material for the organic material used in the organic light emitting device as described above.

### [Prior Art Literature]

### [Patent Literature]

(Patent Literature 1) Korean Unexamined Patent Publication No. 10-2000-0051826

### [DETAILED DESCRIPTION OF THE INVENTION]

### [Technical Problem]

It is an object of the present disclosure to provide a novel organic light emitting material and an organic light emitting device comprising the same.

### [Technical Solution]

Provided herein is a compound represented by the following Chemical Formula 1: 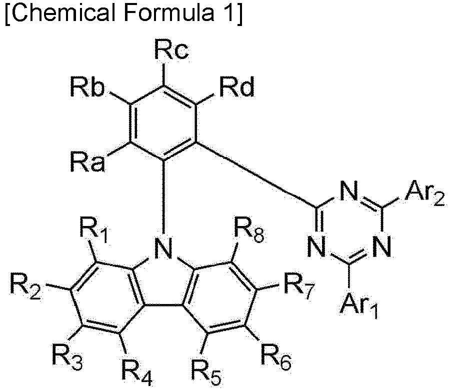 wherein, in Chemical Formula 1,
Ar₁ and Ar₂ are each independently a substituted or unsubstituted C₆₋₆₀ aryl; or a substituted or unsubstituted C₂₋₆₀ heteroaryl containing at least one selected from the group consisting of O and S,
R₁ to R₈ are each independently hydrogen; deuterium; or a substituted or unsubstituted C₆₋₆₀ aryl, with the proviso that at least one of R₁ to R₈ is deuterium, and
Ra to Rd are each independently hydrogen; deuterium; or a C₆₋₆₀ aryl unsubstituted or substituted with deuterium.

Also provided herein is an organic light emitting device comprising: a first electrode; a second electrode that is provided opposite to the first electrode; and one or more organic material layers that are provided between the first electrode and the second electrode, wherein at least one layer of the organic material layers comprises the compound represented by Chemical Formula 1.

### [ADVANTAGEOUS EFFECTS]

The compound represented by Chemical Formula 1 described above can be used as a material of an organic material layer of an organic light emitting device, and can improve the efficiency, achieve low driving voltage and/or improve lifetime characteristics in the organic light emitting device. In particular, the compound represented by Chemical Formula 1 can be used as a hole injection material, hole transport material, light emitting material, electron transport material, and/or electron injection material.

### [BRIEF DESCRIPTION OF THE DRAWINGS]

FIG. 1 shows an example of an organic light emitting device comprising a substrate 1, an anode 2, a light emitting layer 3, and a cathode 4.
FIG. 2 shows an example of an organic light emitting device comprising a substrate 1, an anode 2, a hole injection layer 5, a hole transport layer 6, an electron blocking layer 7, a light emitting layer 3, a hole blocking layer 8, an electron transport layer 9, an electron injection layer 10, and a cathode 4.

### [DETAILED DESCRIPTION OF THE EMBODIMENTS]

Hereinafter, embodiments of the present disclosure will be described to help understanding of the disclosed subject matter.

The present disclosure provides the compound represented by Chemical Formula 1.

In the present disclosure, the notation 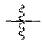 or 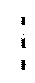 means a bond linked to another substituent group.

In the present disclosure, the term "substituted or unsubstituted" means being unsubstituted or substituted with one or more substituents selected from the group consisting of deuterium; a halogen group; a nitrile group; a nitro group; a hydroxy group; a carbonyl group; an ester group; an imide group; an amino group; a phosphine oxide group; an alkoxy group; an aryloxy group; an alkylthioxy group; an arylthioxy group; an alkylsulfoxy group; an arylsulfoxy group; a silyl group; a boron group; an alkyl group; a cycloalkyl group; an alkenyl group; an aryl group; an aralkyl group; an aralkenyl group; an alkylaryl group; an alkylamine group; an aralkylamine group; a heteroarylamine group; an arylamine group; an arylphosphine group; and a heteroaryl group containing at least one of N, O and S atoms, or being unsubstituted or substituted with a substituent to which two or more substituents of the above-exemplified substituents are linked. For example, "a substituent in which two or more substituents are linked" may be a biphenyl group. Namely, a biphenyl group may be an aryl group, or it may be interpreted as a substituent formed by linking two phenyl groups. In one example, the term "substituted or unsubstituted" may be understood as meaning "being unsubstituted or substituted with one or more substituents selected from the group consisting of deuterium, halogen, a C₁₋₁₀ alkyl, a C₁₋₁₀ alkoxy, a C₆₋₂₀ aryl, and one or more substituents, for example 1 to 5 substituents, selected from the group consisting of a C₂₋₂₀ heteroaryl containing at least one heteroatom of N, O and S". Further, the term "substituted with one or more substituents" as used herein may be understood as meaning "being substituted with 1 to 5 substituents", or "being substituted with one or two substituents".

In the present disclosure, the carbon number of a carbonyl group is not particularly limited, but is preferably 1 to 40. Specifically, it may be a substituent group having the following structure, but is not limited thereto.

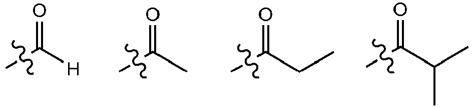

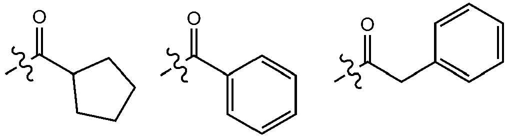

In the present disclosure, an ester group may have a structure in which oxygen of the ester group may be substituted by a straight-chain, branched-chain, or cyclic alkyl group having 1 to 25 carbon atoms, or an aryl group having 6 to 25 carbon atoms. Specifically, the ester group may be a substituent group having the following structural formulas, but is not limited thereto.

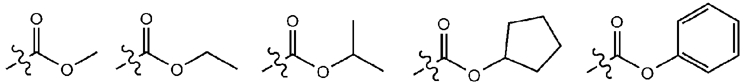

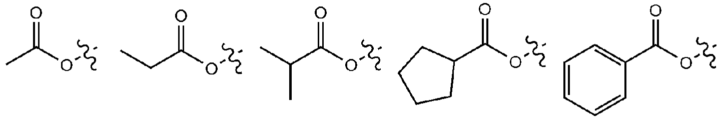

In the present disclosure, the carbon number of an imide group is not particularly limited, but is preferably 1 to 25. Specifically, the imide group may be a substituent group having the following structural formulas, but is not limited thereto.

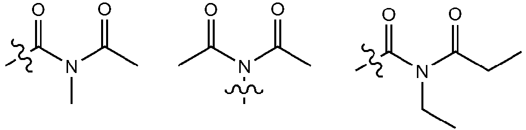

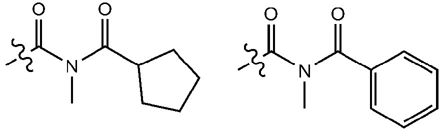

In the present disclosure, specific examples of the silyl group include a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group and the like, but are not limited thereto.

In the present disclosure, a boron group specifically includes a trimethylboron group, a triethylboron group, a t-butyldimethylboron group, a triphenylboron group, and a phenylboron group, but is not limited thereto.

In the present disclosure, examples of a halogen group include fluorine, chlorine, bromine, or iodine.

In the present disclosure, the alkyl group may be straight-chain or branched-chain, and the carbon number thereof is not particularly limited, but is preferably 1 to 40. According to one embodiment, the carbon number of the alkyl group is 1 to 20. According to another embodiment, the carbon number of the alkyl group is 1 to 10. According to another embodiment, the carbon number of the alkyl group is 1 to 6. Specific examples of the alkyl group include methyl, ethyl, propyl, n-propyl, isopropyl, butyl, n-butyl, isobutyl, tert-butyl, sec-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, n-nonyl, 2,2-dimethylheptyl, 1-ethyl-propyl, 1,1-dimethyl-propyl, isohexyl, 2-methylpentyl, 4-methylhexyl, 5-methylhexyl, and the like, but are not limited thereto.

In the present disclosure, the alkenyl group may be straight-chain or branched-chain, and the carbon number thereof is not particularly limited, but is preferably 2 to 40. According to one embodiment, the carbon number of the alkenyl group is 2 to 20. According to another embodiment, the carbon number of the alkenyl group is 2 to 10. According to still another embodiment, the carbon number of the alkenyl group is 2 to 6. Specific examples thereof include vinyl, 1-propenyl, isopropenyl, 1-butenyl, 2-butenyl, 3-butenyl, 1-pentenyl, 2-pentenyl, 3-pentenyl, 3-methyl-1-butenyl, 1,3-butadienyl, allyl, 1-phenylvinyl-1-yl, 2-phenylvinyl-1-yl, 2,2-diphenylvinyl-1-yl, 2-phenyl-2-(naphthyl-1-yl)vinyl-1-yl, 2,2-bis(diphenyl-1-yl)vinyl-1-yl, a stilbenyl group, a styrenyl group, and the like, but are not limited thereto.

In the present disclosure, of the cycloalkyl group is not particularly limited, but the carbon number thereof is preferably 3 to 60. According to another embodiment, the carbon number of the cycloalkyl group is 3 to 30. According to another embodiment, the carbon number of the cycloalkyl group is 3 to 20. According to another embodiment, the carbon number of the cycloalkyl group is 3 to 6. Specific examples thereof include cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, cyclooctyl, and the like, but are not limited thereto.

In the present disclosure, an aryl group is not particularly limited, but the carbon number thereof is preferably 6 to 60, and it may be a monocyclic aryl group or a polycyclic aryl group. According to one embodiment, the carbon number of the aryl group is 6 to 30. According to one embodiment, the carbon number of the aryl group is 6 to 20. The aryl group may be a phenyl group, a biphenyl group, a terphenyl group or the like as the monocyclic aryl group, but is not limited thereto. The polycyclic aryl group includes a naphthyl group, an anthracenyl group, a phenanthrenyl group, a pyrenyl group, a perylenyl group, a chrysenyl group, or the like, but is not limited thereto.

In the present disclosure, the fluorenyl group may be substituted, and two substituent groups may be linked with each other to form a spiro structure. In the case where the fluorenyl group is substituted, 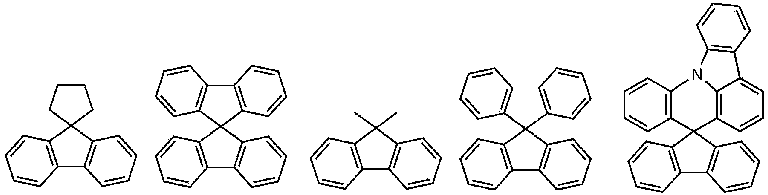 and the like can be formed. However, the structure is not limited thereto.

In the present disclosure, a heteroaryl group is a heteroaryl group containing one or more of O, N, Si and S as a heteroatom, and the carbon number thereof is not particularly limited, but is preferably 2 to 60. According to one embodiment, the carbon number of the heteroaryl group is 6 to 30. According to one embodiment, the carbon number of the heteroaryl group is 6 to 20. Examples of the heteroaryl group include a thiophene group, a furan group, a pyrrole group, an imidazole group, a thiazole group, an oxazol group, an oxadiazol group, a triazol group, a pyridyl group, a bipyridyl group, a pyrimidyl group, a triazine group, an acridyl group, a pyridazine group, a pyrazinyl group, a quinolinyl group, a quinazoline group, a quinoxalinyl group, a phthalazinyl group, a pyridopyrimidinyl group, a pyridopyrazinyl group, a pyrazinopyrazinyl group, an isoquinoline group, an indole group, a carbazole group, a benzoxazole group, a benzoimidazole group, a benzothiazol group, a benzocarbazole group, a benzothiophene group, a dibenzothiophene group, a benzofuranyl group, a phenanthroline group, an isoxazolyl group, a thiadiazolyl group, a phenothiazinyl group, a dibenzofuranyl group, and the like, but are not limited thereto.

In the present disclosure, the aryl group in the aralkyl group, the aralkenyl group, the alkylaryl group and the arylamine group is the same as the examples of the aryl group as defined above. In the present disclosure, the alkyl group in the aralkyl group, the alkylaryl group and the alkylamine group is the same as the examples of the alkyl group as defined above. In the present disclosure, the heteroaryl in the heteroarylamine can be applied to the description of the heteroaryl as defined above. In the present disclosure, the alkenyl group in the aralkenyl group is the same as the examples of the alkenyl group as defined above. In the present disclosure, the description of the aryl group as defined above may be applied except that the arylene is a divalent group. In the present disclosure, the description of the heteroaryl as defined above can be applied except that the heteroarylene is a divalent group. In the present disclosure, the description of the aryl group or cycloalkyl group as defined above can be applied except that the hydrocarbon ring is not a monovalent group but formed by combining two substituent groups. In the present disclosure, the description of the heteroaryl as defined above can be applied, except that the heteroaryl is not a monovalent group but formed by combining two substituent groups.

Preferably, Ar₁ and Ar₂ may be each independently a substituted or unsubstituted C₆₋₂₀ aryl; or a substituted or unsubstituted C₂₋₂₀ heteroaryl containing at least one selected from the group consisting of O and S,
More preferably, Ar₁ and Ar₂ may be each independently phenyl, biphenylyl, dibenzofuranyl, or dibenzothiophenyl, wherein the Ar₁ and Ar₂ may be each independently unsubstituted or substituted with at least one deuterium.

Preferably, at least one of Ar₁ and Ar₂ may be phenyl unsubstituted or substituted with at least one deuterium.

R₁ to R₈ are each independently hydrogen; deuterium; or a substituted or unsubstituted C₆₋₂₀ aryl, with the proviso that at least one of R₁ to R₈ may be deuterium.

More preferably, R₁ to R₈ may be each independently hydrogen, deuterium, or phenyl substituted with five deuterium, with the proviso that least one of R₁ to R₈ may be deuterium.

More preferably, R₁ to R₈ may be each independently deuterium, or phenyl substituted with five deuterium, with the proviso that least one of R₁ to R₈ may be deuterium.

Preferably, four or more of R₁ to R₈ may be deuterium. More preferably, six or more of R₁ to R₈ may be deuterium. Most preferably, seven or more of R₁ to R₈ may be deuterium.

Preferably, Ra to Rd may be each independently hydrogen; deuterium; or a C₆₋₂₀ aryl unsubstituted or substituted with deuterium.

More preferably, Ra to Rd may be each independently hydrogen, deuterium, phenyl, biphenylyl, terphenylyl, naphthyl, anthracenyl, phenanthrenyl, or triphenylenyl, wherein the phenyl, biphenylyl, terphenylyl, naphthyl, anthracenyl, phenanthrenyl, or triphenylenyl may be each independently unsubstituted or substituted with at least one deuterium.

More preferably, Ra to Rd may be each independently hydrogen, phenyl, biphenylyl, terphenylyl, naphthyl, anthracenyl, phenanthrenyl, or triphenylenyl, wherein the phenyl, biphenylyl, terphenylyl, naphthyl, anthracenyl, phenanthrenyl, or triphenylenyl may be each independently unsubstituted or substituted with at least one deuterium.

More preferably, any one of Ra to Rd may be phenyl, biphenylyl, terphenylyl, naphthyl, anthracenyl, phenanthrenyl, or triphenylenyl, wherein the phenyl, biphenylyl, terphenylyl, naphthyl, anthracenyl, phenanthrenyl, and triphenylenyl may be each independently unsubstituted or substituted with at least one deuterium, and the rest may be hydrogen.

Most preferably, any one of Ra to Rd may be any one selected from the group consisting of the following, and the rest may be hydrogen:

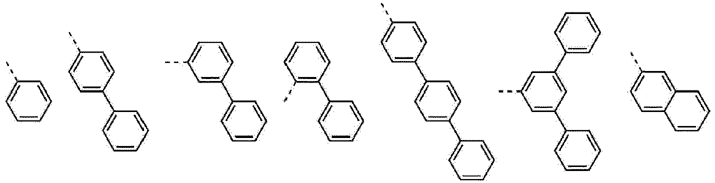

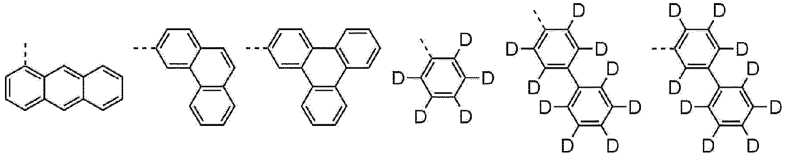

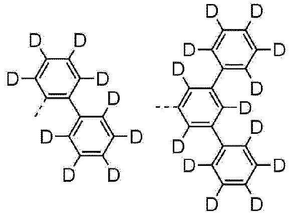

Representative examples of the compound represented by Chemical Formula 1 are as follows:

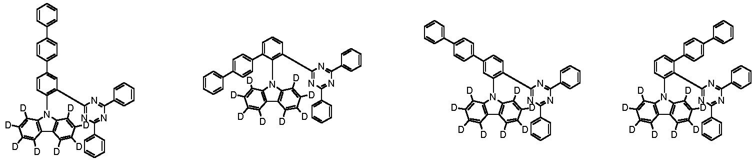

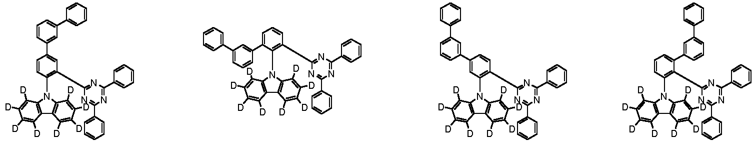

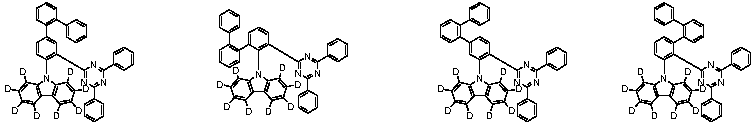

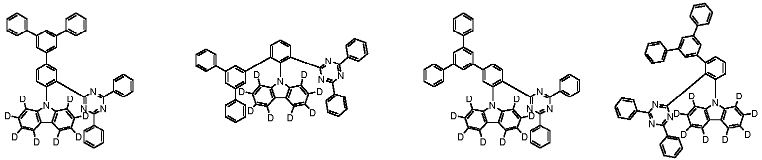

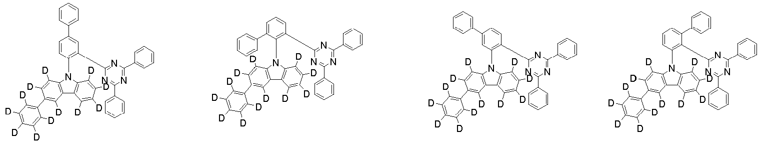

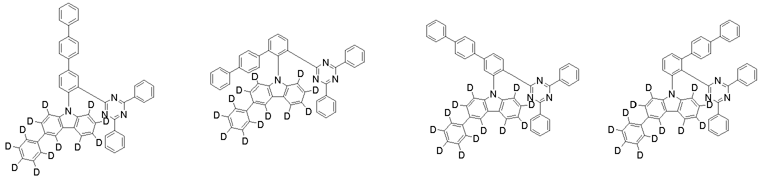

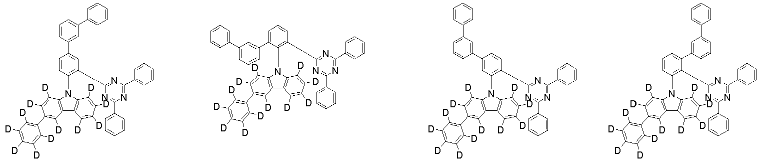

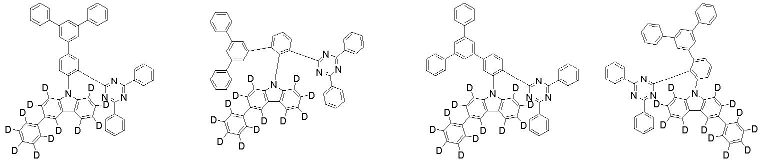

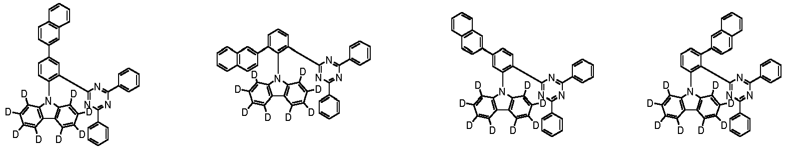

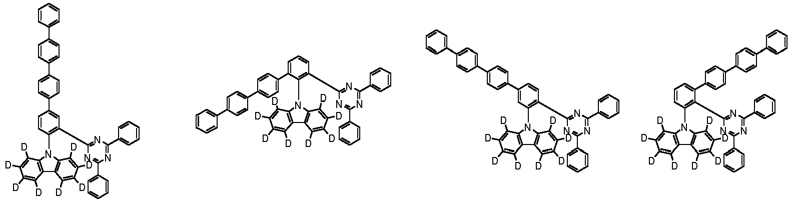

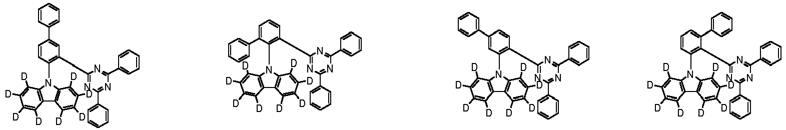

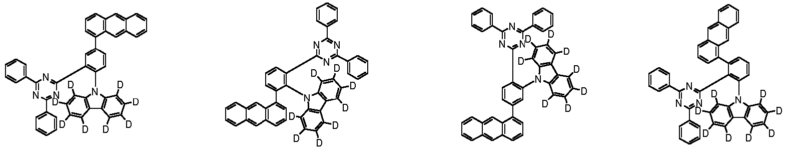

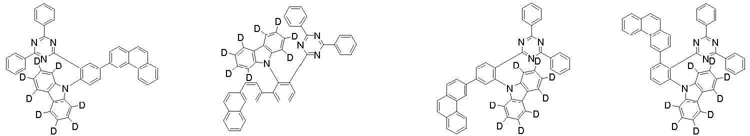

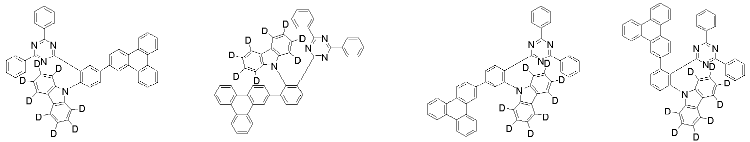

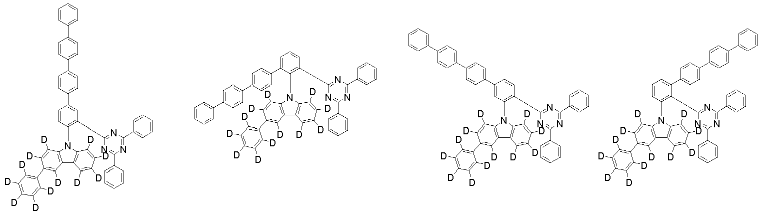

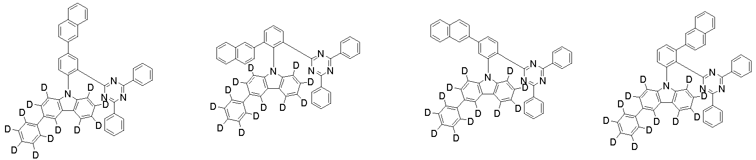

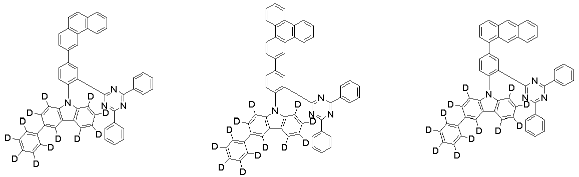

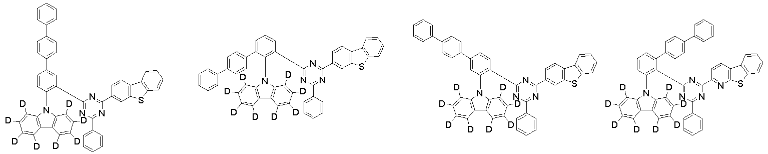

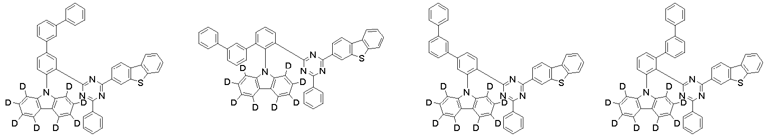

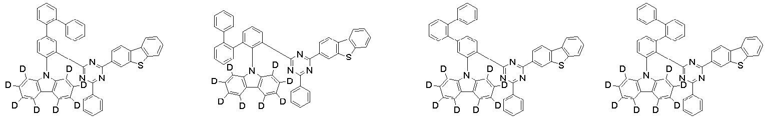

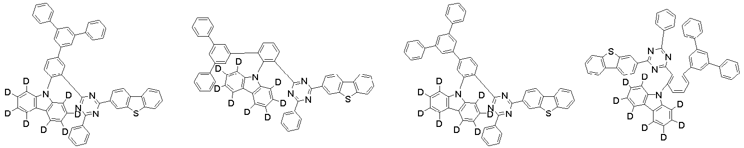

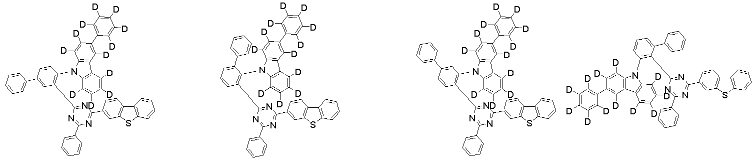

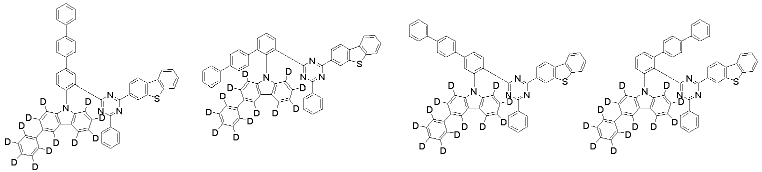

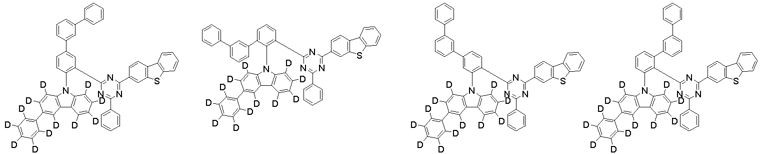

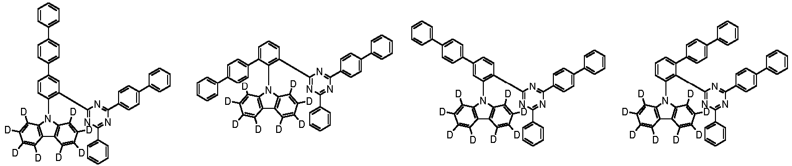

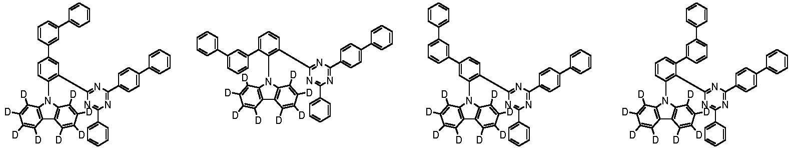

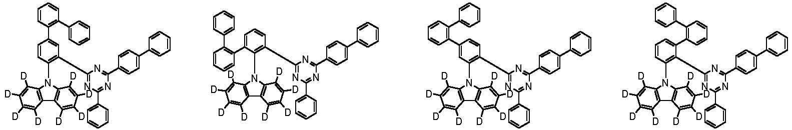

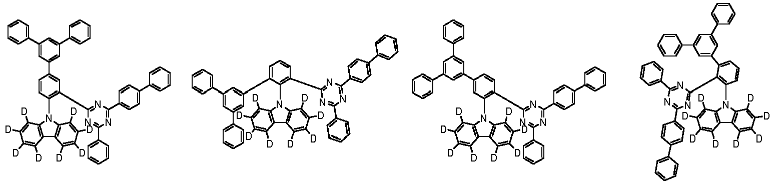

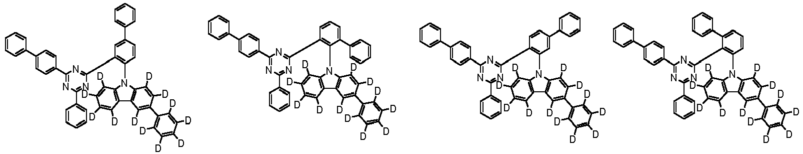

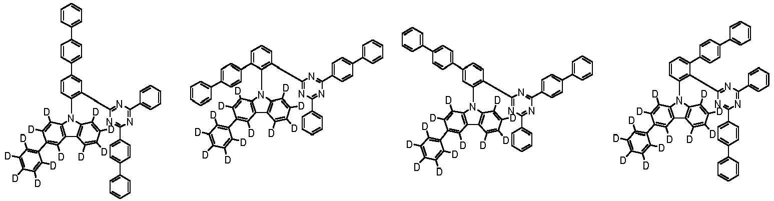

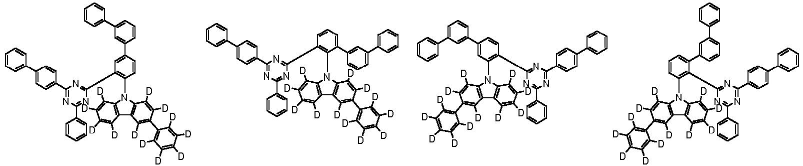

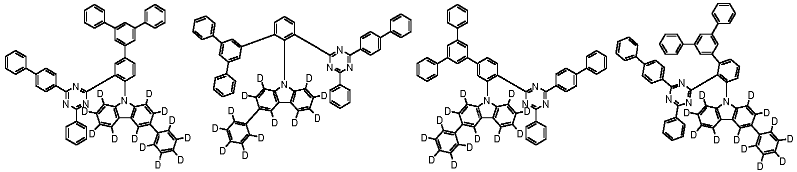

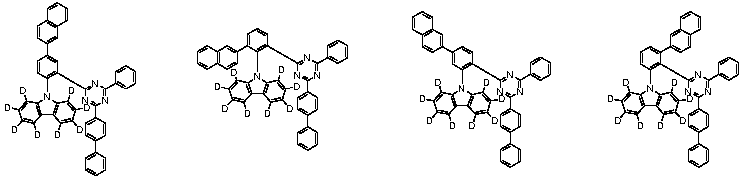

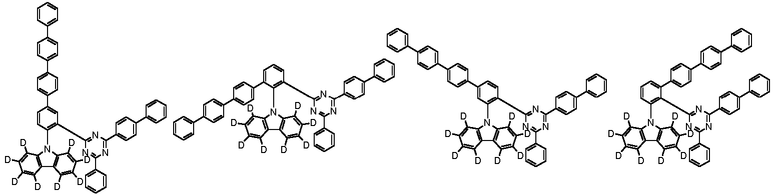

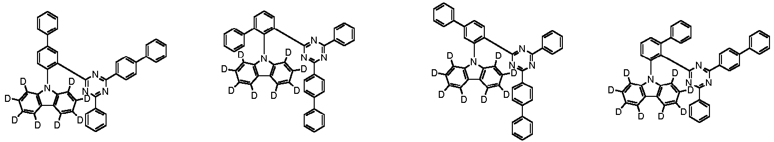

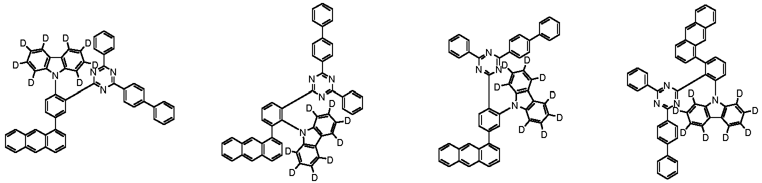

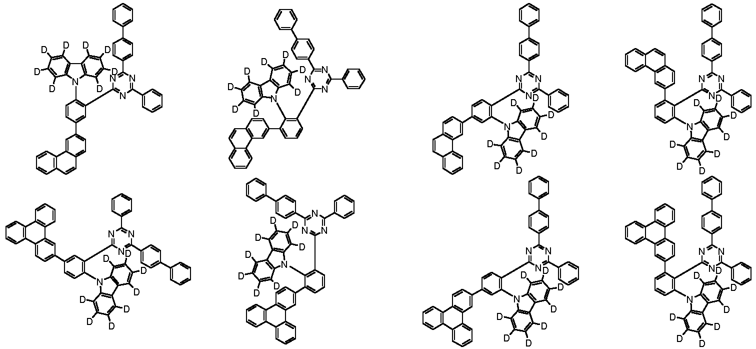

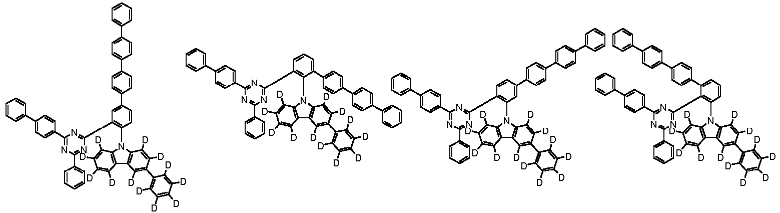

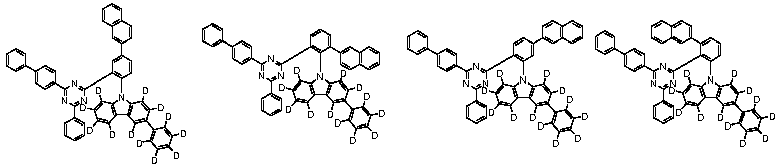

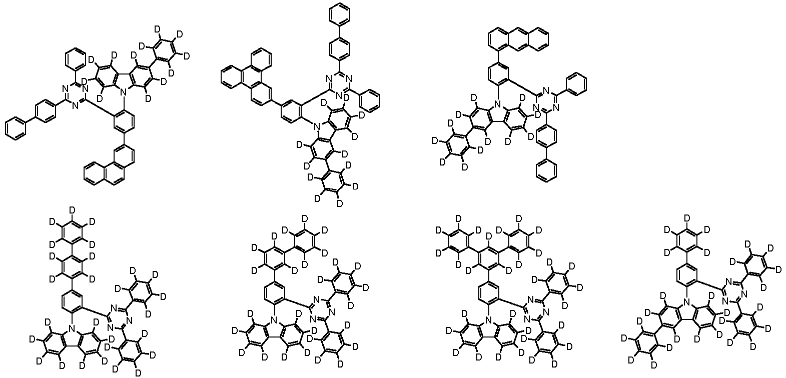

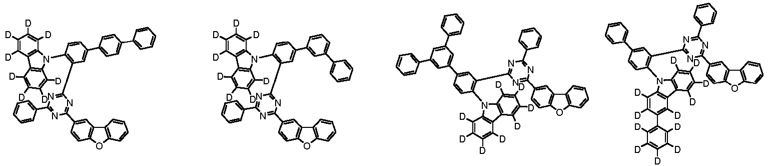

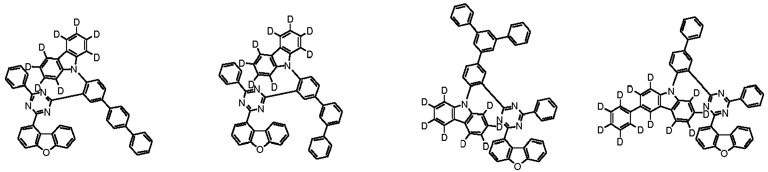

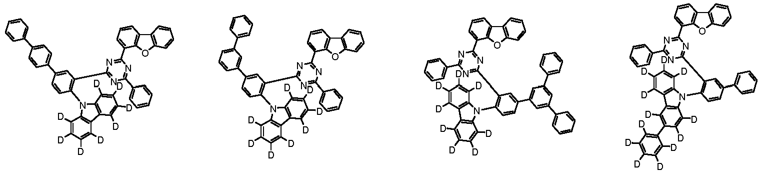

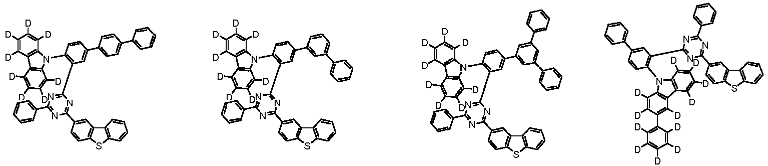

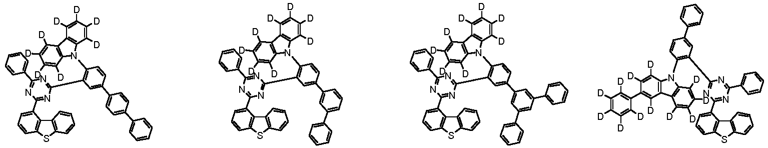

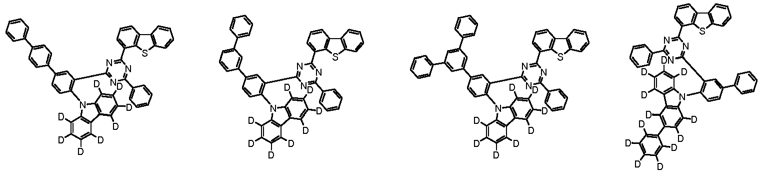

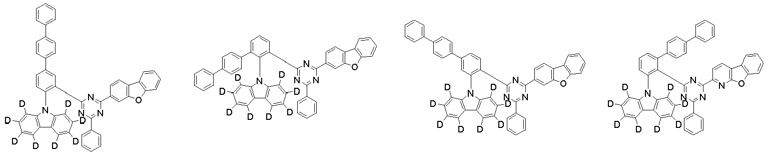

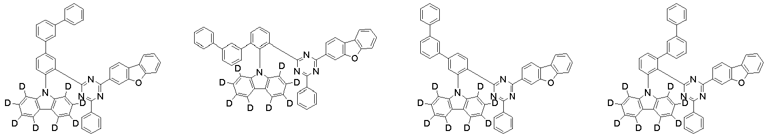

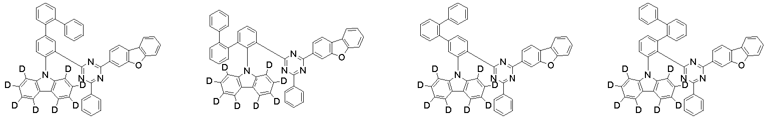

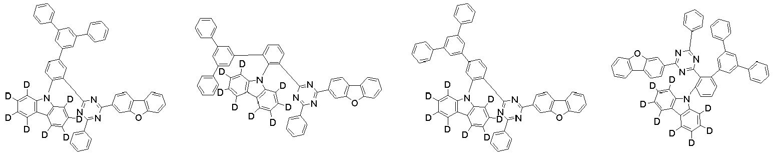

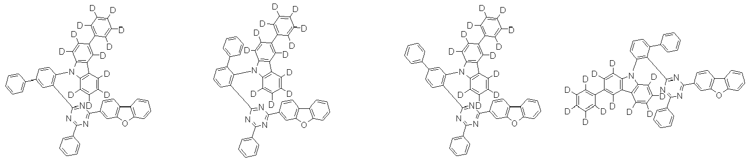

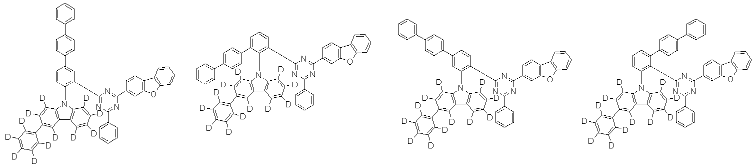

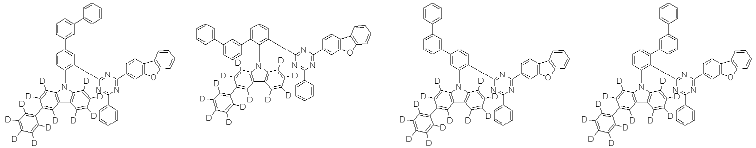

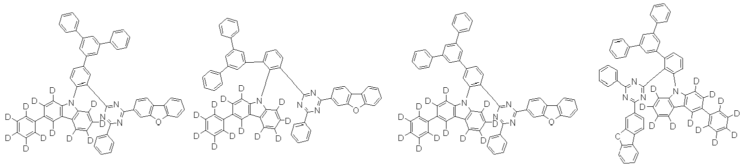

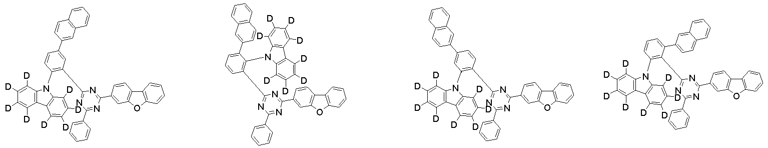

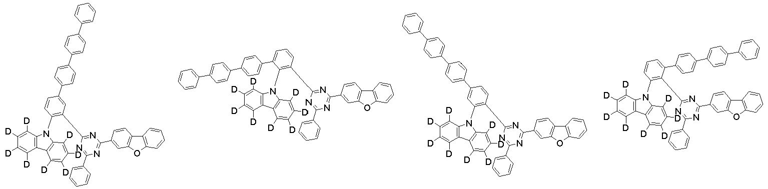

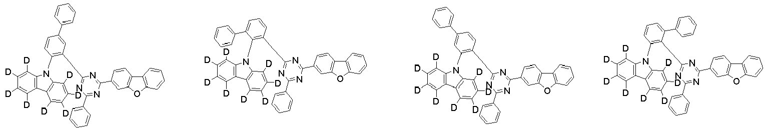

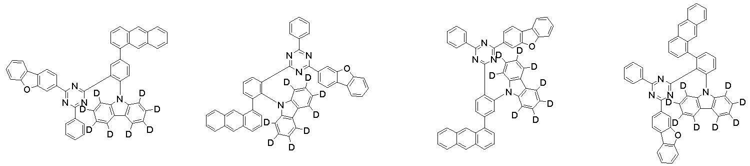

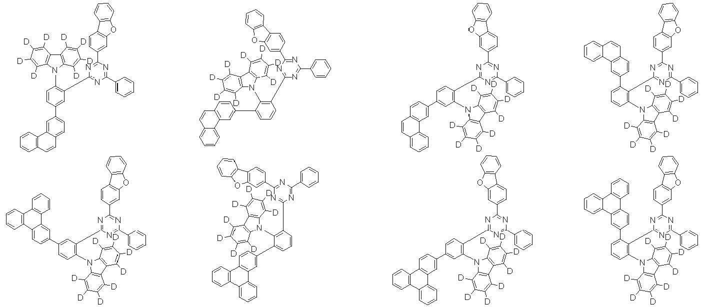

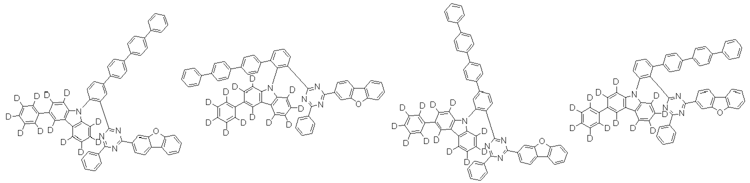

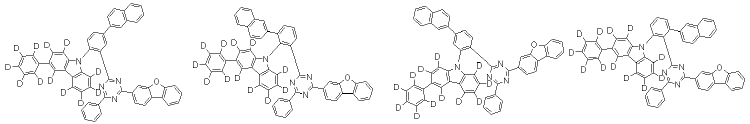

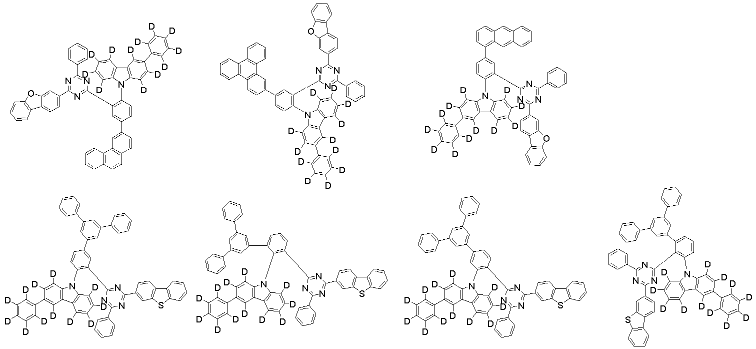

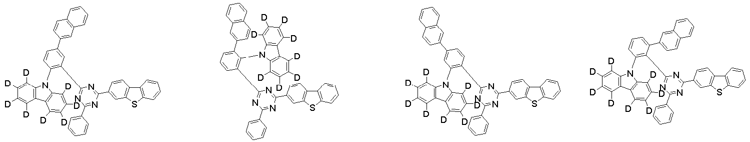

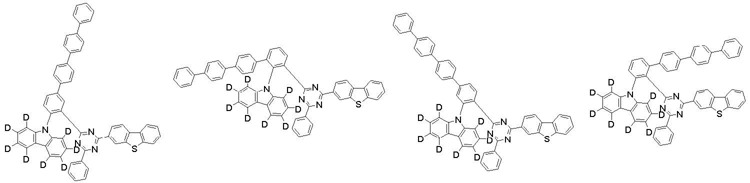

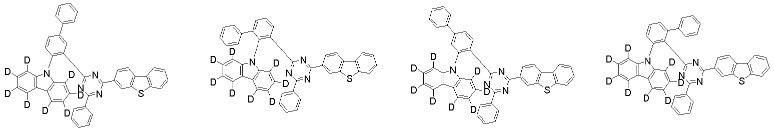

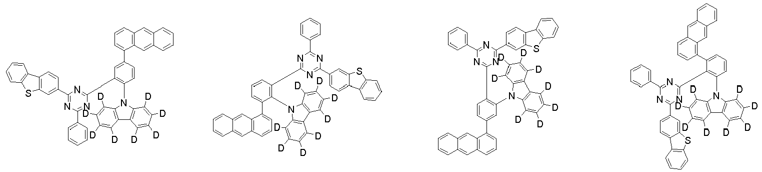

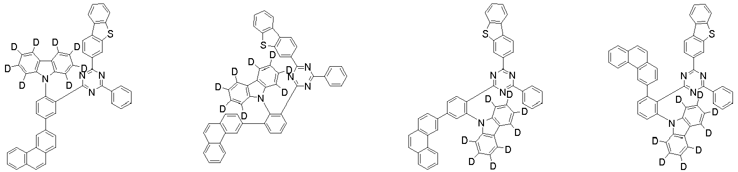

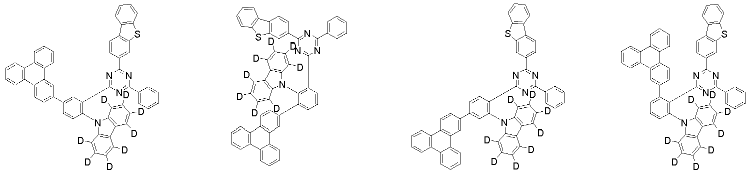

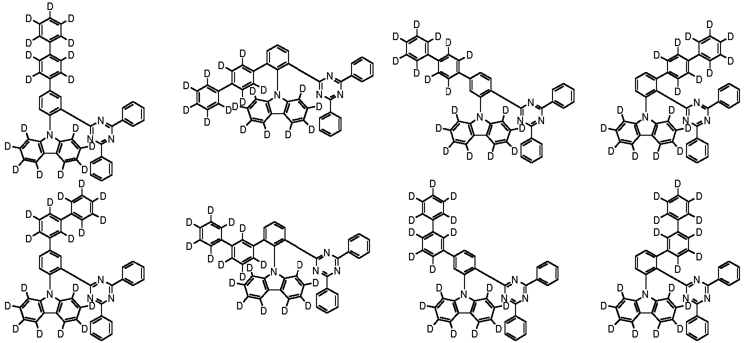

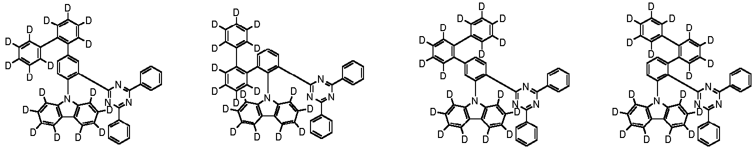

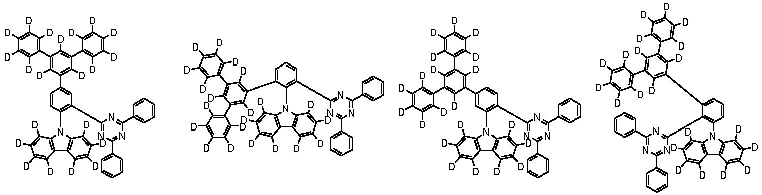

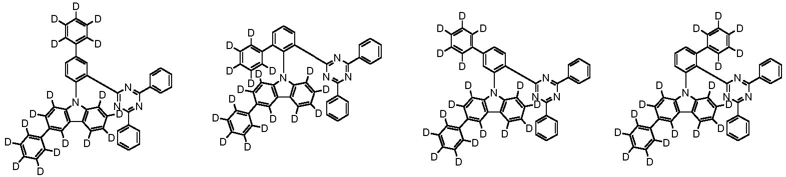

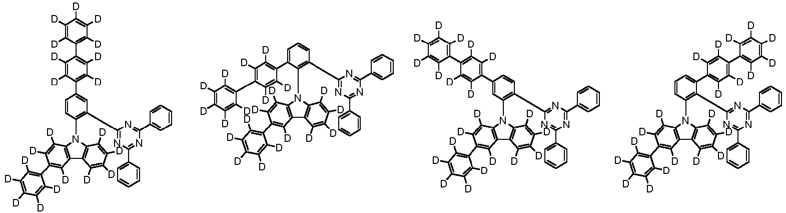

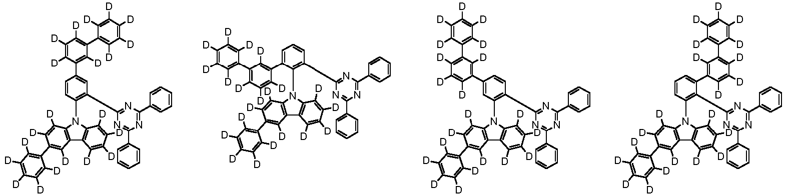

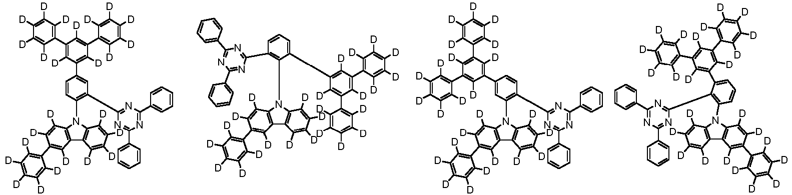

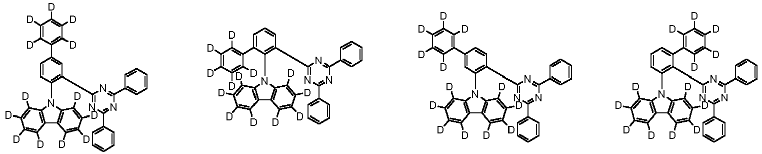

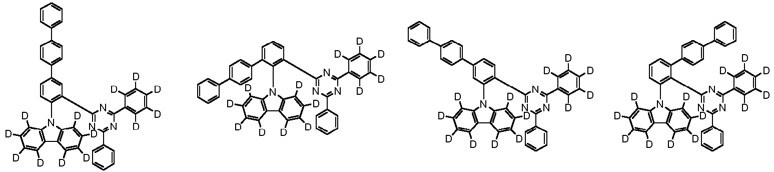

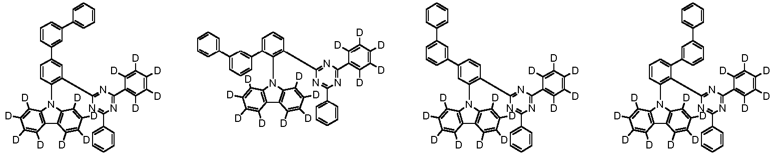

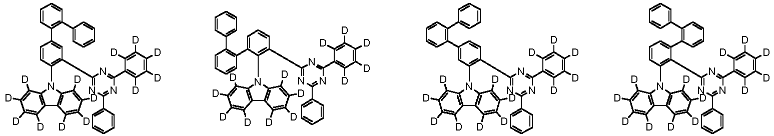

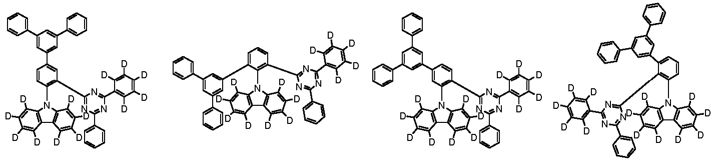

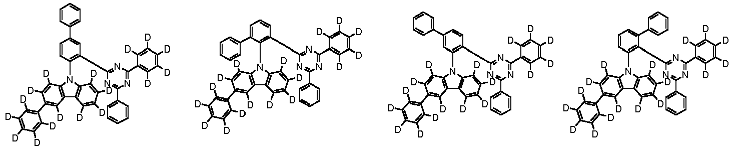

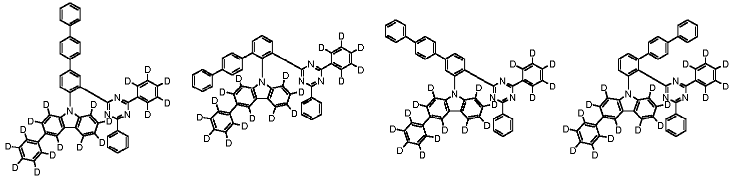

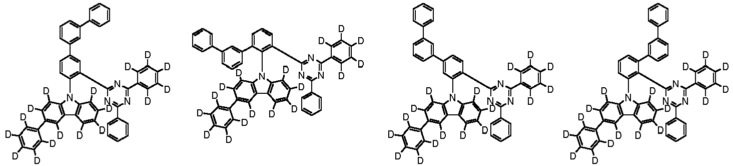

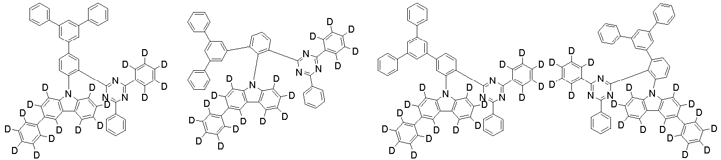

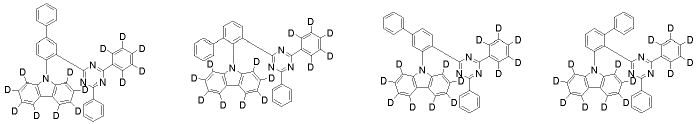

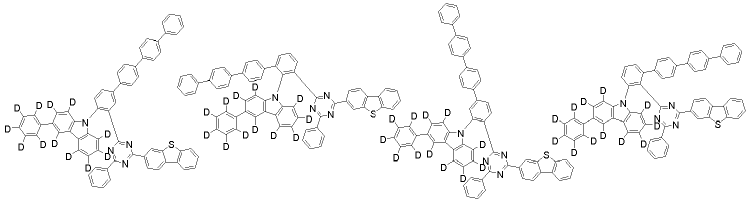

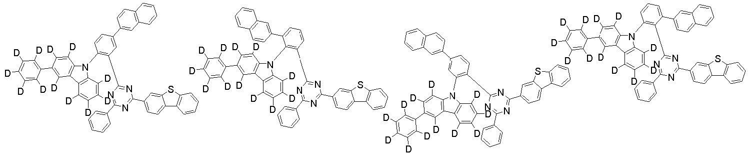

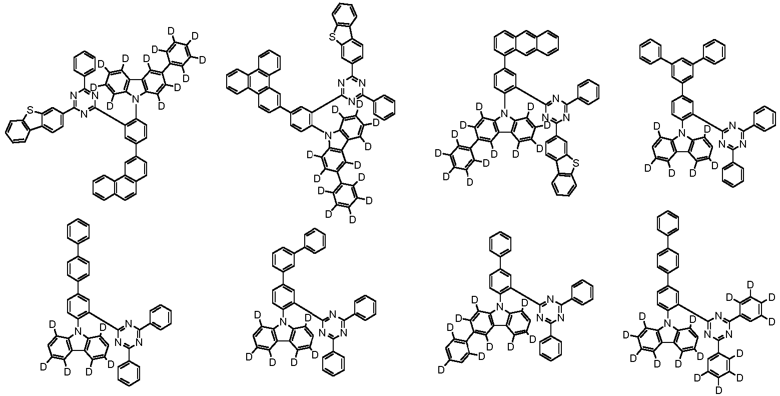

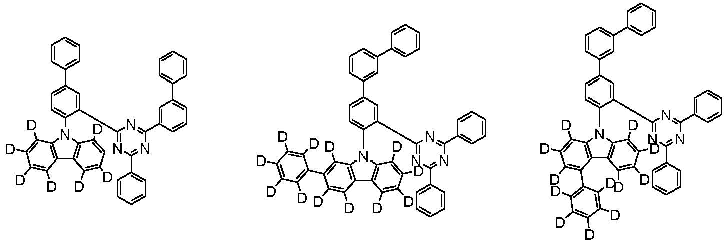

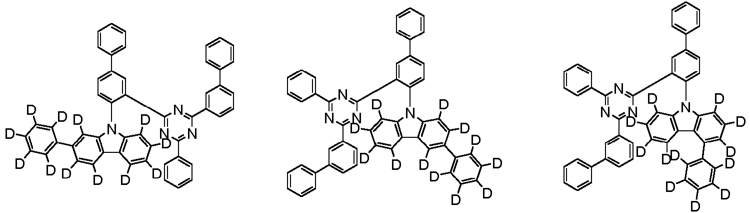

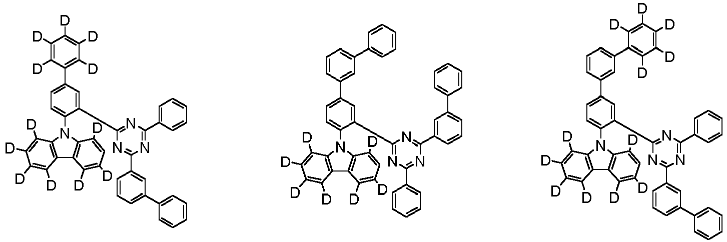

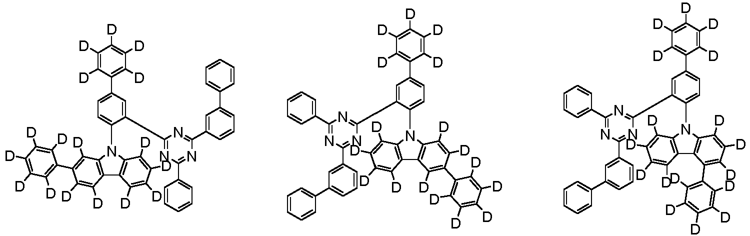

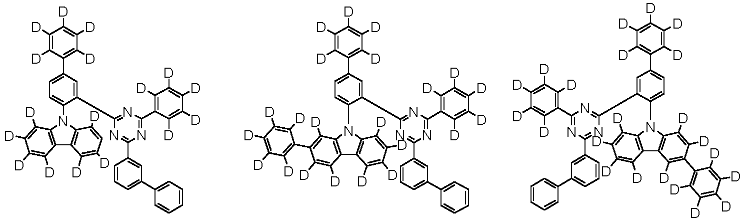

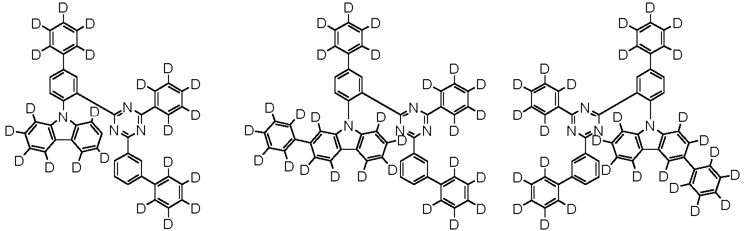

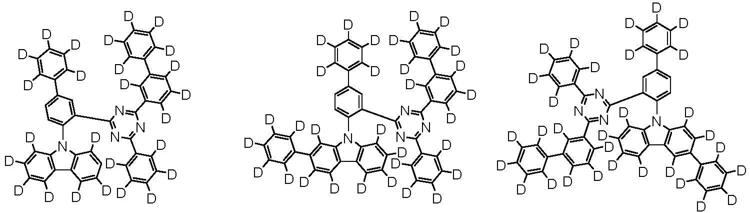

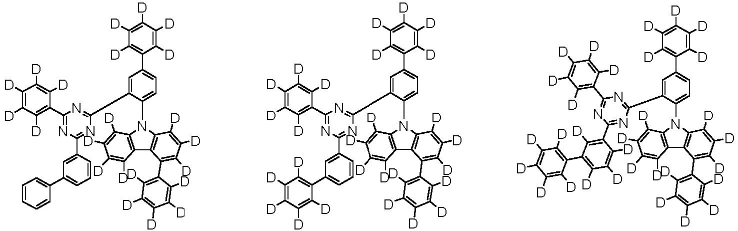

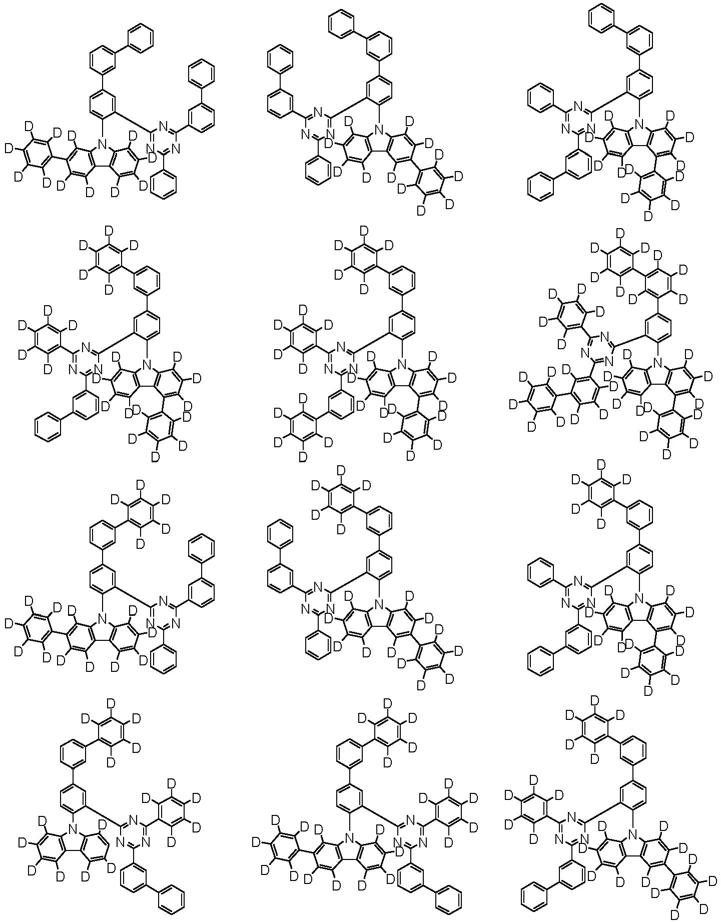

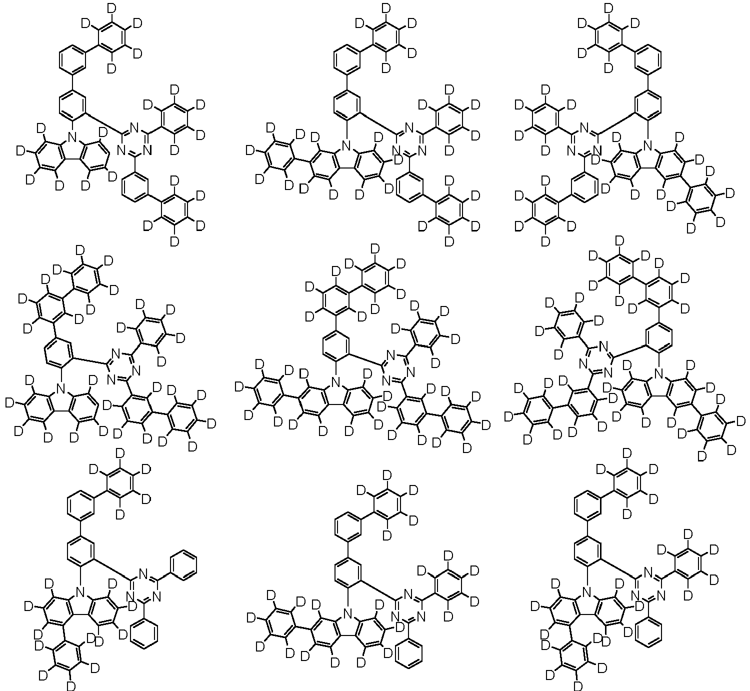

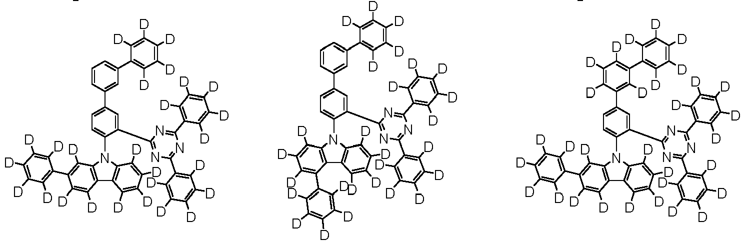

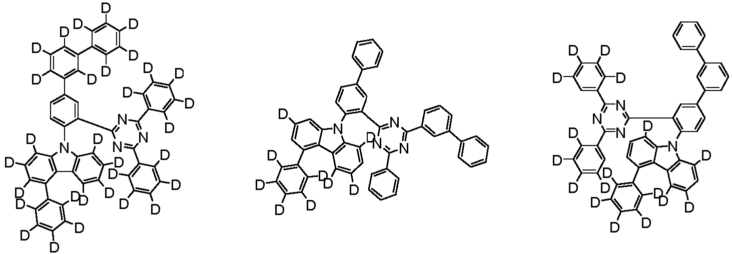

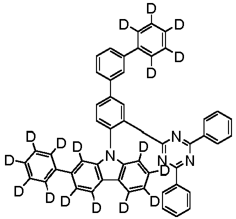

The compound represented by Chemical Formula 1 can be prepared by a preparation method as shown in the following Reaction Scheme 1 as an example, and other remaining compounds can also be prepared in a similar manner. 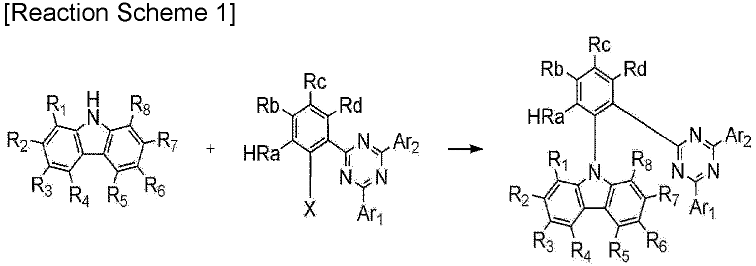 in Reaction Scheme 1, R₁ to R₈, Ra to Rd, Ar₁ and Ar₂ are as defined in Chemical Formula 1, and X is halogen, preferably X is chloro or bromo.

Reaction Scheme 1 is an amine substitution reaction, which is preferably carried out in the presence of a palladium catalyst and a base, and a reactive group for the amine substitution reaction can be modified as known in the art. The preparation method can be further embodied in Preparation Examples described hereinafter.

Further, according to the present disclosure, there is provided an organic light emitting device comprising a compound represented by Chemical Formula 1. In one example, the present disclosure provides an organic light emitting device comprising: a first electrode; a second electrode that is provided opposite to the first electrode; and one or more organic material layers that are provided between the first electrode and the second electrode, wherein at least one layer of the organic material layers includes the compound represented by Chemical Formula 1.

The organic material layer of the organic light emitting device of the present disclosure may have a single-layer structure, or it may have a multilayered structure in which two or more organic material layers are stacked. For example, the organic light emitting device of the present disclosure may have a structure comprising a hole injection layer, a hole transport layer, an electron blocking layer, a light emitting layer, a hole blocking layer, an electron transport layer, an electron injection layer and the like as the organic material layer. However, the structure of the organic light emitting device is not limited thereto, and it may include a smaller number of organic layers.

Further, the organic material layer may include a light emitting layer, wherein the organic material layer may include the compound represented by Chemical Formula 1.

Further, the organic material layer may include a hole transport layer, a hole injection layer, or a layer that simultaneously performs hole transport and hole injection, wherein the hole transport layer, the hole injection layer, or the layer that simultaneously performs hole transport and hole injection may include the compound represented by Chemical Formula 1.

Further, the organic material layer may include an electron transport layer, an electron injection layer, or an electron injection and transport layer, wherein the electron transport layer, the electron injection layer, or the electron injection and transport layer may include the compound represented by Chemical Formula 1.

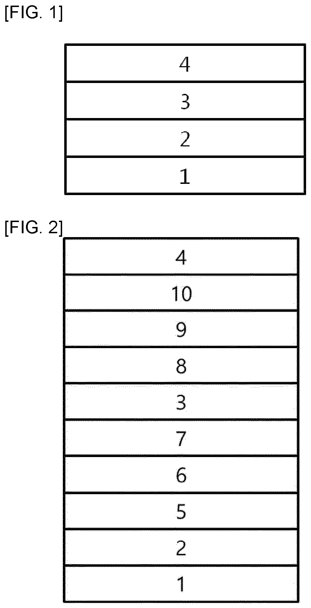

Further, the organic light emitting device according to the present disclosure may be a normal type organic light emitting device in which an anode, one or more organic material layers, and a cathode are sequentially stacked on a substrate. Further, the organic light emitting device according to the present disclosure may be an inverted type organic light emitting device in which a cathode, one or more organic material layers, and an anode are sequentially stacked on a substrate. For example, the structure of the organic light emitting device according to an embodiment of the present disclosure is illustrated in FIGS. 1 and 2.

FIG. 1 shows an example of an organic light emitting device comprising a substrate 1, an anode 2, a light emitting layer 3, and a cathode 4. FIG. 2 shows an example of an organic light emitting device comprising a substrate 1, an anode 2, a hole injection layer 5, a hole transport layer 6, an electron blocking layer 7, a light emitting layer 3, a hole blocking layer 8, an electron transport layer 9, an electron injection layer 10, and a cathode 4.

In such a structure, the compound represented by Chemical Formula 1 may be included in the light emitting layer.

The organic light emitting device according to the present disclosure may be manufactured by materials and methods known in the art, except that at least one of the organic material layers includes the compound represented by Chemical Formula 1. Further, when the organic light emitting device includes a plurality of organic material layers, the organic material layers may be formed of the same material or different materials.

For example, the organic light emitting device according to the present disclosure can be manufactured by sequentially stacking a first electrode, an organic material layer and a second electrode on a substrate. In this case, the organic light emitting device may be manufactured by depositing a metal, metal oxides having conductivity, or an alloy thereof on the substrate using a PVD (physical vapor deposition) method such as a sputtering method or an e-beam evaporation method to form an anode, forming organic material layers including the hole injection layer, the hole transport layer, the light emitting layer and the electron transport layer thereon, and then depositing a material that can be used as the cathode thereon. In addition to such a method, the organic light emitting device can be manufactured by sequentially depositing a cathode material, an organic material layer and an anode material on a substrate.

Further, the compound represented by Chemical Formula 1 can be formed into an organic layer by a solution coating method as well as a vacuum deposition method at the time of manufacturing an organic light emitting device. Wherein, the solution coating method means a spin coating, a dip coating, a doctor blading, an inkjet printing, a screen printing, a spray method, a roll coating, or the like, but is not limited thereto.

In addition to such a method, the organic light emitting device may be manufactured by sequentially depositing a cathode material, an organic material layer and an anode material on a substrate (International Publication WO2003/012890). However, the manufacturing method is not limited thereto.

In one example, the first electrode is an anode, and the second electrode is a cathode, or alternatively, the first electrode is a cathode and the second electrode is an anode.

As the anode material, generally, a material having a large work function is preferably used so that holes can be smoothly injected into the organic material layer. Specific examples of the anode material include metals such as vanadium, chrome, copper, zinc, and gold, or an alloy thereof; metal oxides such as zinc oxides, indium oxides, indium tin oxides (ITO), and indium zinc oxides (IZO); a combination of metals and oxides, such as ZnO:Al or SnO₂:Sb; conductive polymers such as poly(3-methylthiophene), poly[3,4-(ethylene-1,2-dioxy)thiophene](PEDOT), polypyrrole, and polyaniline, and the like, but are not limited thereto.

As the cathode material, generally, a material having a small work function is preferably used so that electrons can be easily injected into the organic material layer. Specific examples of the cathode material include metals such as magnesium, calcium, sodium, potassium, titanium, indium, yttrium, lithium, gadolinium, aluminum, silver, tin, and lead, or an alloy thereof; a multilayered structure material such as LiF/Al or LiO₂/Al, and the like, but are not limited thereto.

The hole injection layer is a layer for injecting holes from the electrode, and the hole injection material is preferably a compound which has a capability of transporting the holes, thus has a hole injecting effect in the anode and an excellent hole injecting effect to the light emitting layer or the light emitting material, prevents excitons produced in the light emitting layer from moving to a hole injection layer or the electron injection material, and further is excellent in the ability to form a thin film. It is preferable that a HOMO (highest occupied molecular orbital) of the hole injection material is between the work function of the anode material and a HOMO of a peripheral organic material layer. Specific examples of the hole injection material include metal porphyrin, oligothiophene, an arylamine-based organic material, a hexanitrilehexaazatriphenylene-based organic material, a quinacridone-based organic material, a perylene-based organic material, anthraquinone, polyaniline and polythiophene-based conductive polymer, and the like, but are not limited thereto.

The hole transport layer is a layer that receives holes from a hole injection layer and transports the holes to the light emitting layer. The hole transport material is suitably a material having large mobility to the holes, which may receive holes from the anode or the hole injection layer and transfer the holes to the light emitting layer. Specific examples thereof include an arylamine-based organic material, a conductive polymer, a block copolymer in which a conjugate portion and a non-conjugate portion are present together, and the like, but are not limited thereto.

The electron blocking layer means a layer provided between the hole transport layer and the light emitting layer in order to prevent the electrons injected in the cathode from being transferred to the hole transport layer without being recombined in the light emitting layer, which may also be referred to as an electron inhibition layer or an electron stopping layer. The electron blocking layer is preferably a material having the smaller electron affinity than the electron transport layer.

The light emitting material is preferably a material which may receive holes and electrons transported from a hole transport layer and an electron transport layer, respectively, and combine the holes and the electrons to emit light in a visible ray region, and has good quantum efficiency to fluorescence or phosphorescence. Specific examples of the light emitting material include an 8-hydroxy-quinoline aluminum complex(Alq₃); a carbazole-based compound; a dimerized styryl compound; BAlq; a 10-hydroxybenzoquinoline-metal compound; a benzoxazole, benzthiazole and benzimidazole-based compound; a poly(p-phenylenevinylene)(PPV)-based polymer; a spiro compound; polyfluorene, lubrene, and the like, but are not limited thereto.

The light emitting layer may include a host material and a dopant material. The host material includes a fused aromatic ring derivative, a heterocycle-containing compound, or the like. Specific examples of the fused aromatic ring derivatives include anthracene derivatives, pyrene derivatives, naphthalene derivatives, pentacene derivatives, phenanthrene compounds, fluoranthene compounds, and the like. Examples of the heterocyclic-containing compounds include carbazole derivatives, dibenzofuran derivatives, ladder-type furan compounds, pyrimidine derivatives, and the like, but are not limited thereto.

Examples of the dopant material include an aromatic amine derivative, a styrylamine compound, a boron complex, a fluoranthene compound, a metal complex, and the like. Specifically, the aromatic amine derivative is a substituted or unsubstituted fused aromatic ring derivative having an arylamino group, and examples thereof include pyrene, anthracene, chrysene, periflanthene and the like, which have an arylamino group. The styrylamine compound is a compound where at least one arylvinyl group is substituted in substituted or unsubstituted arylamine, in which one or two or more substituent groups selected from the group consisting of an aryl group, a silyl group, an alkyl group, a cycloalkyl group and an arylamino group are substituted or unsubstituted. Specific examples thereof include styrylamine, styryldiamine, styryltriamine, styryltetramine, and the like, but are not limited thereto. Further, the metal complex includes an iridium complex, a platinum complex, and the like, but is not limited thereto.

The hole blocking layer is a layer provided between the electron transport layer and the light emitting layer in order to prevent the holes injected in the anode from being transferred to the electron transport layer without being recombined in the light emitting layer, which may also be referred to as a hole inhibition layer or a hole stopping layer. The hole blocking layer is preferably a material having the large ionization energy.

The electron transport layer is a layer that may receive the electrons from the electron injection layer and transport the electrons to the light emitting layer, and an electron transport material is suitably a material which may receive well injection of electrons from a cathode and transfer the electrons to a light emitting layer, and has a large mobility for electrons. Specific examples of the electron transport material include: an Al complex of 8-hydroxyquinoline; a complex including Alqs; an organic radical compound; a hydroxyflavone-metal complex, and the like, but are not limited thereto. The electron transport layer may be used with any desired cathode material, as used according to a conventional technique. In particular, appropriate examples of the cathode material are a typical material which has a low work function, followed by an aluminum layer or a silver layer. Specific examples thereof include cesium, barium, calcium, ytterbium, and samarium, in each case followed by an aluminum layer or a silver layer.

The electron injection layer is a layer which injects electrons from an electrode, and is preferably a compound which has a capability of transporting electrons, has an effect of injecting electrons from a cathode and an excellent effect of injecting electrons into a light emitting layer or a light emitting material, prevents excitons produced from the light emitting layer from moving to a hole injection layer, and is also excellent in the ability to form a thin film. Specific examples of the electron injection layer include fluorenone, anthraquinodimethane, diphenoquinone, thiopyran dioxide, oxazole, oxadiazole, triazole, imidazole, perylenetetracarboxylic acid, fluorenylidene methane, anthrone, and the like, and derivatives thereof, a metal complex compound, a nitrogen-containing 5-membered ring derivative, and the like, but are not limited thereto.

Examples of the metal complex compound include 8-hydroxyquinolinato lithium, bis(8-hydroxyquinolinato)zinc, bis(8-hydroxyquinolinato)copper, bis(8-hydroxyquinolinato)manganese, tris(8-hydroxyquinolinato)aluminum, tris(2-methyl-8-hydroxyquinolinato)aluminum, tris(8-hydroxyquinolinato)gallium, bis(10-hydroxybenzo[h]quinolinato)beryllium, bis(10-hydroxybenzo[h]quinolinato)zinc, bis(2-methyl-8-quinolinato)chlorogallium, bis(2-methyl-8-quinolinato)(o-cresolato)gallium, bis(2-methyl-8-quinolinato)(1-naphtholato)aluminum, bis(2-methyl-8-quinolinato)(2-naphtholato)gallium, and the like, but are not limited thereto.

Meanwhile, in the present disclosure, the "electron injection and transport layer" is a layer that performs both the roles of the electron injection layer and the electron transport layer, and the materials that perform the roles of each layer may be used alone or stacked and used in combination, but are not limited thereto.

The organic light emitting device according to the present disclosure may be a bottom emission type device, a top emission type device, or a double side emission type device, and in particular, it may be a bottom emission type light emitting device that requires relatively high luminous efficiency.

In addition, the compound represented by Chemical Formula 1 may be included in an organic solar cell or an organic transistor in addition to an organic light emitting device.

Below, embodiments are described in more detail to assist in the understanding of the present disclosure. However, the following Examples are for illustrative purposes only, and are not intended to limit the scope of the present disclosure.

### [PREPARATION EXAMPLE]

### Preparation Example A: Preparation of Compound A1

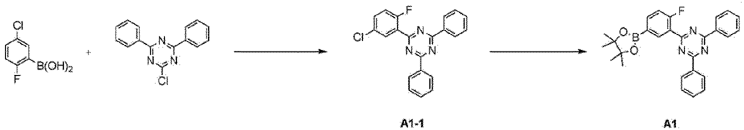

### 1) Preparation of Compound A 1-1

(5-Chloro-2-fluorophenyl)boronic acid(40 g, 229.4 mmol) and 2-chloro-4,6-diphenyl-1,3,5-triazine(61.4 g, 229.4 mmol) were added to 400 ml of tetrahydrofuran under a nitrogen atmosphere, and the mixture was stirred and refluxed. Then, potassium carbonate(95.1 g, 688.2mmol) was dissolved in 95 ml of water, added thereto, sufficiently stirred and then [1,1'-bis(diphenylphosphino)ferrocene]dichloropalladium(II) (3.4 g, 4.6 mmol) was added. After the reaction for 11 hours, the reaction mixture was cooled to room temperature, the organic layer and the aqueous layer were separated, and then the organic layer was distilled. This was again added to and dissolved in 830 mL of toluene, washed twice with water, and the organic layer was separated. Anhydrous magnesium sulfate was added thereto, stirred, filtered, and the filtrate was distilled under reduced pressure. The concentrated compound was purified by a silica column using toluene and ethyl acetate to prepare Compound A1-1 as a grey solid (58.9g, 71%, MS: [M+H]+ = 362.8)

### 2) Preparation of Compound A1

A1-1 (50 g, 138.2 mmol) and bis(pinacolato)diboron (38.7 g, 165.8 mmol) were added to 750ml of Diox under a nitrogen atmosphere, and the mixture was stirred and refluxed. Then, potassium acetate (39.9 g, 414.6 mmol) was added thereto, sufficiently stirred, and then palladium dibenzylideneacetone palladium (2.4 g, 4.1 mmol) and tricyclohexylphosphine (2.3 g, 8.3 mmol) were added. After the reaction for 5 hours, the reaction mixture was cooled to room temperature, the organic layer was filtered to remove salt, the filtered organic layer was distilled. This was again added to and dissolved in 626 mL of toluene, washed twice with water, and the organic layer was separated. Anhydrous magnesium sulfate was added thereto, stirred, filtered, and the filtrate was distilled under reduced pressure. The concentrated compound was purified by a silica column using chloroform and ethyl acetate to prepare Compound A1 as a yellow solid (53.3 g, 85%, MS: [M+H]+ = 454.3).

### Preparation Example B: Preparation of Compound B1

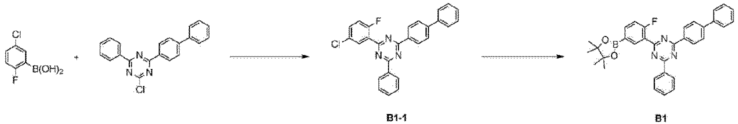

Compound B1 (35.3 g, 73%, MS: [M+H]+ = 530.4) was prepared in the same manner as in the preparation method of Compound A1, except that in Preparation Example A, 2-([1,1'-biphenyl]-4-yl)-4-chloro-6-phenyl-1,3,5-triazine was used instead of 2-chloro-4,6-diphenyl-1,3,5-triazine.

### Preparation Example C: Preparation of Compound C1

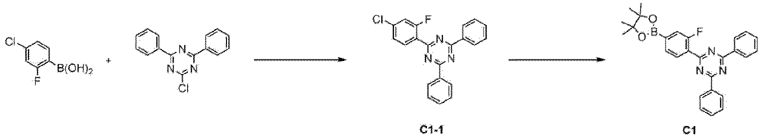

### 1) Preparation of Compound C1-1

(4-Chloro-2-fluorophenyl)boronic acid(40 g, 229.4 mmol) and 2-chloro-4,6-diphenyl-1,3,5-triazine(61.4 g, 229.4 mmol) were added to 400 ml of tetrahydrofuran under a nitrogen atmosphere, and the mixture was stirred and refluxed. Then, potassium carbonate(95.1 g, 688.2mmol) was dissolved in 95 ml of water, added thereto, sufficiently stirred and then [1,1'-bis(diphenylphosphino)ferrocene]dichloropalladium(II) (3.4 g, 4.6 mmol) was added. After the reaction for 6 hours, the reaction mixture was cooled to room temperature, the organic layer and the aqueous layer were separated, and then the organic layer was distilled. This was again added to and dissolved in 830 mL of toluene, washed twice with water, and the organic layer was separated. Anhydrous magnesium sulfate was added thereto, stirred, filtered, and the filtrate was distilled under reduced pressure. The concentrated compound was purified by a silica column using toluene and ethyl acetate to prepare Compound C1-1 as a grey solid (66.4 g, 80%, MS: [M+H]+ = 362.8).

### 2) Preparation of Compound C1

C1-1 (40 g, 110.6 mmol) and bis(pinacolato)diboron (30.9 g, 132.7 mmol) were added to 600 ml of Diox under a nitrogen atmosphere, and the mixture was stirred and refluxed. Then, potassium acetate (31.9 g, 331.7 mmol) was added thereto, sufficiently stirred, and then palladiumdibenzylideneacetonepalladium (1.9 g, 3.3 mmol) and tricyclohexylphosphine (1.9 g, 6.6 mmol) were added. After the reaction for 6 hours, the reaction mixture was cooled to room temperature, the organic layer was filtered to remove salt, and then the filtered organic layer was distilled. This was again added to and dissolved in 501 mL of chloroform, washed twice with water, and the organic layer was separated. Anhydrous magnesium sulfate was added thereto, stirred, filtered, and the filtrate was distilled under reduced pressure. The concentrated compound was purified by a silica column using chloroform and ethyl acetate to prepare Compound C1 as a grey solid (31.6 g, 63%, MS: [M+H]+ = 454.3).

### Preparation Example D: Preparation of Compound D1

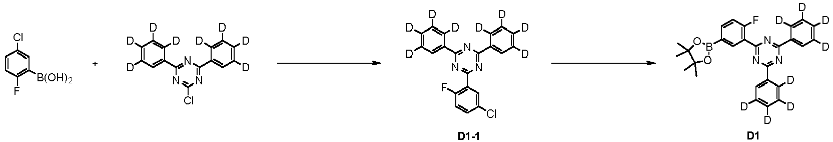

Compound D1 (30.5 g, 70%, MS: [M+H]+ = 461.2) was prepared in the same manner as in the preparation method of Compound A1, except that in Preparation Example A, 2-chloro-4,6-bis(phenyl-2,3,4,5-d4)-1,3,5-triazine was used instead of 2-chloro-4,6-diphenyl-1,3,5-triazine.

### Preparation Example E: Preparation of Compound E1

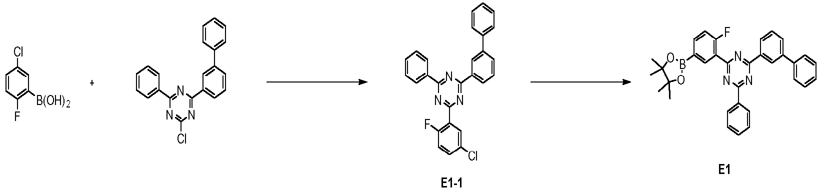

Compound E1 (35.8 g, 77%, MS: [M+H]+ = 529) was prepared in the same manner as in the preparation method of Compound A1, except that in Preparation Example A, 2-([1,1'-biphenyl]-3-yl)-4-chloro-6-phenyl-1,3,5-triazine was used instead of 2-chloro-4,6-diphenyl-1,3,5-triazine.

### Preparation Example 1: Preparation of Compound 1

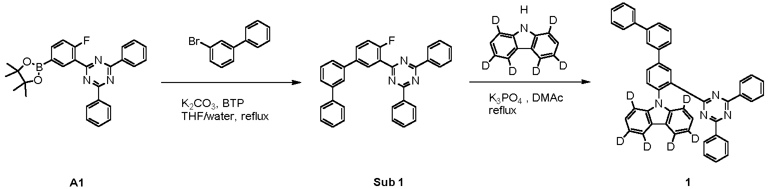

### Step 1) Preparation of Compound Sub 1

A1(36 g, 79.4 mmol) and 3-bromo-1,1'-biphenyl(18.5 g, 79.4 mmol) were added to 720 ml of tetrahydrofuran under a nitrogen atmosphere, and the mixture was stirred and refluxed. Then, potassium carbonate(32.9 g, 238.2 mmol) was dissolved in 33 ml of water, added thereto, sufficiently stirred, and then bis(tri-tert-butylphosphine)palladium(0.8 g, 1.6 mmol) was added. After the reaction for 3 hours, the reaction mixture was cooled to room temperature, the resulting solid was filtered. The solid was added to and dissolved in 1904 mL of chloroform, washed twice with water, and the organic layer was separated. Anhydrous magnesium sulfate was added thereto, stirred, filtered, and the filtrate was distilled under reduced pressure. The concentrated compound was purified by a silica column using chloroform and ethyl acetate to prepare Compound Sub 1 as a grey solid (28.9 g, 76%, MS: [M+H]+ = 480.6).

### Step 2) Preparation of Compound 1

Compound Sub 1(40 g, 83.4 mmol) and 9H-carbazole-1,3,4,5,6,8-d6(14.5 g, 83.4 mmol) were added to 320 ml of dimethylacetamide under a nitrogen atmosphere, and the mixture was stirred and refluxed. Then, potassium phosphate tribasic(53.1 g, 250.2 mmol) was added thereto, sufficiently stirred and then reacted for 1 hour. The reaction mixture was cooled to room temperature, the organic layer was filtered to remove salt, and then the filtered organic layer was distilled. This was again added to and dissolved in 1583 mL of chloroform, washed twice with water, and the organic layer was separated. Anhydrous magnesium sulfate was added thereto, stirred, filtered, and the filtrate was distilled under reduced pressure. The concentrated compound was purified by a silica column using chloroform and ethyl acetate to prepare Compound 1 as a yellow solid (30.1 g, 57%, MS: [M+H]+ = 633.8).

### Preparation Example 2: Preparation of Compound 2

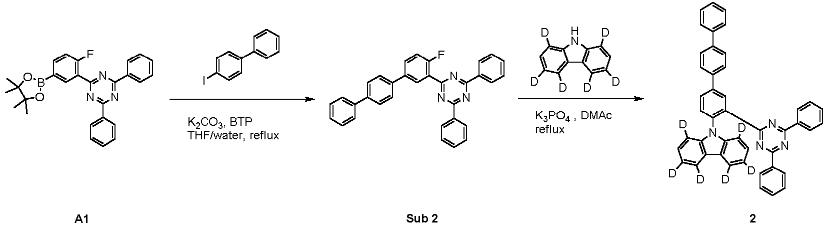

### Step 1) Preparation of Compound Sub 2

Compound A1 (36 g, 79.4 mmol) and 4-iodo-1,1'-biphenyl(22.2 g, 79.4 mmol) were added to 720 ml of tetrahydrofuran under a nitrogen atmosphere, and the mixture was stirred and refluxed. Then, potassium carbonate(32.9 g, 238.2 mmol) was dissolved in 33 ml of water, added thereto, sufficiently stirred, and then bis(tri-tert-butylphosphine)palladium(0.8 g, 1.6 mmol) was added. After the reaction for 4 hours, the reaction mixture was cooled to room temperature, the resulting solid was filtered. The solid was added to and dissolved in 1904 mL of chloroform, washed twice with water, and the organic layer was separated. Anhydrous magnesium sulfate was added thereto, stirred, filtered, and the filtrate was distilled under reduced pressure. The concentrated compound was purified by a silica column using chloroform and ethyl acetate to prepare Compound Sub 2 as a red solid (34.7 g, 91%, MS: [M+H]+ = 480.6).

### Step 2) Preparation of Compound 2

Compound Sub 2(40 g, 83.4 mmol) and 9H-carbazole-1,3,4,5,6,8-d6(14.5 g, 83.4 mmol) were added to 320 ml of dimethylacetamide under a nitrogen atmosphere, and the mixture was stirred and refluxed. Then, potassium phosphate tribasic (53.1 g, 250.2 mmol) was added thereto, sufficiently stirred and then reacted for 1 hour. The reaction mixture was cooled to room temperature, the organic layer was filtered to remove salt, and then the filtered organic layer was distilled. This was again added to and dissolved in 1583 mL of chloroform, washed twice with water, and the organic layer was separated. Anhydrous magnesium sulfate was added thereto, stirred, filtered, and the filtrate was distilled under reduced pressure. The concentrated compound was purified by a silica column using chloroform and ethyl acetate to prepare Compound 2 as a yellow solid (41.7 g, 79%, MS: [M+H]+ = 633.8).

### Preparation Example 3: Preparation of Compound 3

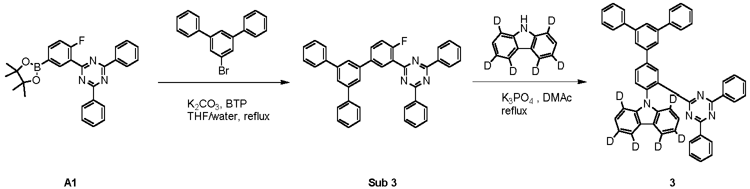

### Step 1) Preparation of Compound Sub 3

Compound A1(36 g, 79.4 mmol) and 5'-bromo-1,1':3',1"-terphenyl(24.6 g, 79.4 mmol) were added to 720 ml of tetrahydrofuran under a nitrogen atmosphere, and the mixture was stirred and refluxed. Then, potassium carbonate(32.9 g, 238.2 mmol) was dissolved in 33 ml of water, added thereto, sufficiently stirred, and then bis(tri-tert-butylphosphine)palladium(0.8 g, 1.6 mmol) was added. After the reaction for 3 hours, the reaction mixture was cooled to room temperature, the resulting solid was filtered. The solid was added to and dissolved in 2206 mL of chloroform, washed twice with water, and the organic layer was separated. Anhydrous magnesium sulfate was added thereto, stirred, filtered, and the filtrate was distilled under reduced pressure. The concentrated compound was purified by a silica column using chloroform and ethyl acetate to prepare Compound Sub 3 as a grey solid (27.4 g, 62%, MS: [M+H]+ = 556.7).

### Step 2) Preparation of Compound 3

Compound Sub 3(40 g, 72 mmol) and 9H-carbazole-1,3,4,5,6,8-d6(12.5 g, 72 mmol) were added to 320 ml of dimethylacetamide under a nitrogen atmosphere, and the mixture was stirred and refluxed. Then, potassium phosphate tribasic(45.8 g, 216 mmol) was added thereto, sufficiently stirred and then reacted for 2 hours. The reaction mixture was cooled to room temperature, the organic layer was filtered to remove salt, and then the filtered organic layer was distilled. This was again added to and dissolved in 1531 mL of chloroform, washed twice with water, and the organic layer was separated. Anhydrous magnesium sulfate was added thereto, stirred, filtered, and the filtrate was distilled under reduced pressure. The concentrated compound was purified by a silica column using chloroform and ethyl acetate to prepare Compound 3 as a yellow solid (40.8 g, 80%, MS: [M+H]+ = 709.9).

### Preparation Example 4: Preparation of Compound 4

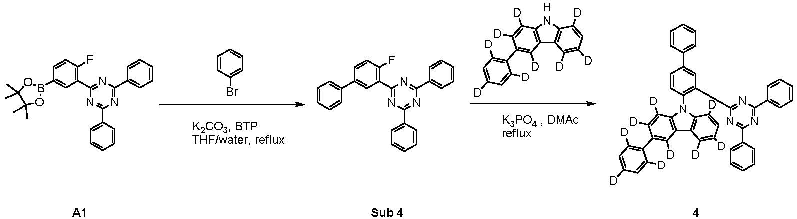

### Step 1) Preparation of Compound Sub 4

Compound A1(30 g, 66.2 mmol) and bromobenzene(10.4 g, 66.2 mmol) were added to 300 ml of tetrahydrofuran under a nitrogen atmosphere, and the mixture was stirred and refluxed. Then, potassium carbonate (27.4 g, 198.5 mmol) was dissolved in 27 ml of water, added thereto, sufficiently stirred, and then bis(tri-tert-butylphosphine)palladium (0.7 g, 1.3 mmol) was added. After the reaction for 3 hours, the reaction mixture was cooled to room temperature, the resulting solid was filtered. The solid was added to and dissolved in 1335 mL of chloroform, washed twice with water, and the organic layer was separated. Anhydrous magnesium sulfate was added thereto, stirred, filtered, and the filtrate was distilled under reduced pressure. The concentrated compound was purified by a silica column using chloroform and ethyl acetate to prepare Compound Sub 4 as a grey solid (19.5 g, 73%, MS: [M+H]+ = 404.5).

### Step 2) Preparation of Compound 4

Compound Sub 4(30 g, 74.4 mmol) and 3-(phenyl-2,4,6-d3)-9H-carbazole-1,2,4,5,6,8-d6(18.8 g, 74.4 mmol) were added to 240 ml of methylacetamide under a nitrogen atmosphere, and the mixture was stirred and refluxed. Then, potassium phosphate tribasic(47.4 g, 223.1 mmol) was added thereto, sufficiently stirred, and then reacted for 1 hour. The reaction mixture was cooled to room temperature, the organic layer was filtered to remove salt, and then the filtered organic layer was distilled. This was again added to and dissolved in 1418 mL of chloroform, washed twice with water, and the organic layer was separated. Anhydrous magnesium sulfate was added thereto, stirred, filtered, and the filtrate was distilled under reduced pressure. The concentrated compound was purified by a silica column using chloroform and ethyl acetate to prepare Compound 4 as a yellow solid (32.1 g, 68%, MS: [M+H]+ = 636.8).

### Preparation Example 5: Preparation of Compound 5

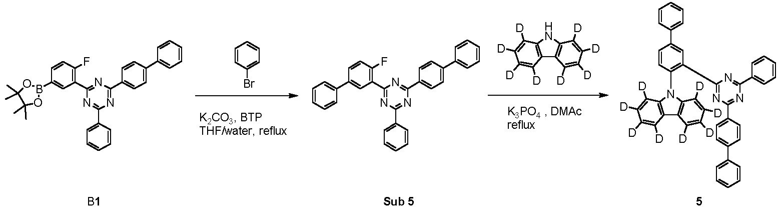

### Step 1) Preparation of Compound Sub 5

Compound B1(30 g, 56.7 mmol) and bromobenzene(8.9 g, 56.7 mmol) were added to 450 ml of tetrahydrofuran under a nitrogen atmosphere, and the mixture was stirred and refluxed. Then, potassium carbonate(23.5 g, 170 mmol) was dissolved in 23 ml of water, added thereto, sufficiently stirred, and then [1,1'-bis(diphenylphosphino)ferrocene]dichloropalladium(II) (0.8 g, 1.1 mmol) was added. After the reaction for 12 hours, the reaction mixture was cooled to room temperature, the organic layer and the aqueous layer were separated, and then the organic layer was distilled. This was again added to and dissolved in 272 mL of toluene, washed twice with water, and the organic layer was separated. Anhydrous magnesium sulfate was added thereto, stirred, filtered, and the filtrate was distilled under reduced pressure. The concentrated compound was purified by a silica column using toluene and ethyl acetate to prepare Compound Sub 5 as a grey solid (16.6 g, 61%, MS: [M+H]+ = 480.6).

### Step 2) Preparation of Compound 5

Compound Sub 5(30 g, 62.6 mmol) and 9H-carbazole-d8 (11 g, 62.6 mmol) were added to 240 ml of dimethylacetamide under a nitrogen atmosphere, and the mixture was stirred and refluxed. Then, potassium phosphate tribasic(39.8 g, 187.7 mmol) was added thereto, sufficiently stirred, and then reacted for 1 hour. The reaction mixture was cooled to room temperature, the organic layer was filtered to remove salt, and then the filtered organic layer was distilled. This was again added to and dissolved in 1191 mL of chloroform, washed twice with water, and the organic layer was separated. Anhydrous magnesium sulfate was added thereto, stirred, filtered, and the filtrate was distilled under reduced pressure. The concentrated compound was purified by a silica column using chloroform and ethyl acetate to prepare Compound 5 as a yellow solid (20.7 g, 52%, MS: [M+H]+ = 635.8).

### Preparation Example 6: Preparation of Compound 6

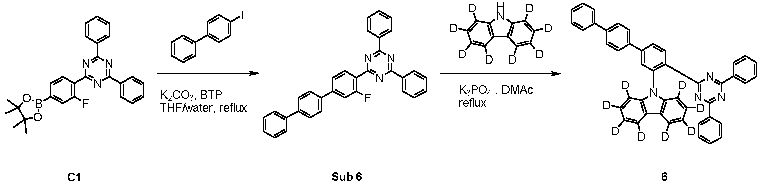

### Step 1) Preparation of Compound Sub 6

Compound C1(35 g, 77.2 mmol) and 4-iodo-1,1'-biphenyl(21.6 g, 77.2 mmol) were added to 350 ml of tetrahydrofuran under a nitrogen atmosphere, and the mixture was stirred and refluxed. Then, potassium carbonate(32 g, 231.6 mmol) was dissolved in 32 ml of water, added thereto, sufficiently stirred, and then bis(tri-tert-butylphosphine)palladium(0.8 g, 1.5 mmol) was added. After the reaction for 4 hours, the reaction mixture was cooled to room temperature, the resulting solid was filtered. The solid was added to and dissolved in 1851 mL of chloroform, washed twice with water, and the organic layer was separated. Anhydrous magnesium sulfate was added thereto, stirred, filtered, and the filtrate was distilled under reduced pressure. The concentrated compound was purified by a silica column using chloroform and ethyl acetate to prepare Compound Sub 6 as a grey solid (29.3g, 79%, MS: [M+H]+ = 480.6).

### Step 2) Preparation of Compound 6

Compound Sub 6(25 g, 52.1 mmol) and 9H-carbazole-d8(9.1 g, 52.1 mmol) were added to 200 ml of dimethylacetamide under a nitrogen atmosphere, and the mixture was stirred and refluxed. Then, potassium phosphate tribasic(33.2 g, 156.4 mmol) was added thereto, sufficiently stirred, and then reacted for 2 hour. The reaction mixture was cooled to room temperature, the organic layer was filtered to remove salt, and then the filtered organic layer was distilled. This was again added to and dissolved in 993 mL of chloroform, washed twice with water, and the organic layer was separated. Anhydrous magnesium sulfate was added thereto, stirred, filtered, and the filtrate was distilled under reduced pressure. The concentrated compound was purified by a silica column using chloroform and ethyl acetate to prepare Compound 6 as a yellow solid (17.5 g, 53%, MS: [M+H]+ = 635.8).

### Preparation Example 7: Preparation of Compound 7

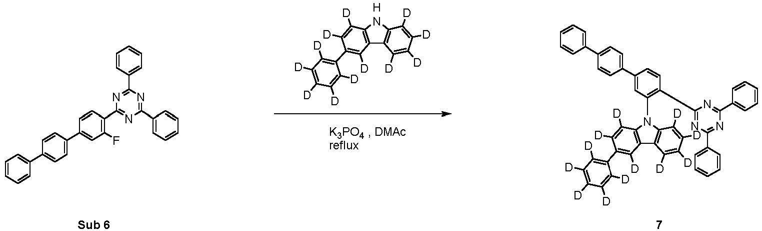

Compound 7 (29.5 g, 66%, MS: [M+H]+ = 715.9) was prepared in the same manner as in the preparation method of Compound 6, except that in Preparation Example 6, 3-(phenyl-d5)-9H-carbazole-1,2,4,5,6,7,8-d7 was used instead of 9H-carbazole-d8.

### Preparation Example 8: Preparation of Compound 8

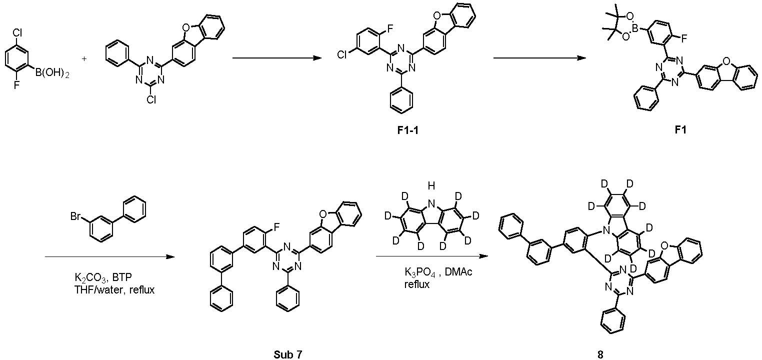

### Step 1) Preparation of Compound F1

Compound F1 (39.4 g, 75%, MS: [M+H]+ = 544.4) was prepared in the same manner as in the preparation method of Compound A1, except that in Preparation Example 1, 2-chloro-4-(dibenzo[b,d]furan-3-yl)-6-phenyl-1,3,5-triazine was used instead of 2-chloro-4,6-diphenyl-1,3,5-triazine.

### Step 2) Preparation of Compound Sub 7

Compound F1(35 g, 64.4 mmol) and 3-bromo-1,1'-biphenyl(15 g, 64.4 mmol) were added to 350 ml of tetrahydrofuran under a nitrogen atmosphere, and the mixture was stirred and refluxed. Then, potassium carbonate(26.7 g, 193.2 mmol) was dissolved in 27 ml of water, added thereto, sufficiently stirred, and then bis(tri-tert-butylphosphine)palladium(0.7 g, 1.3 mmol) was added. After the reaction for 3 hours, the reaction mixture was cooled to room temperature, the resulting solid was filtered. The solid was added to and dissolved in 1834 mL of chloroform, washed twice with water, and the organic layer was separated. Anhydrous magnesium sulfate was added thereto, stirred, filtered, and the filtrate was distilled under reduced pressure. The concentrated compound was purified by a silica column using chloroform and ethyl acetate to prepare Compound Sub 7 as a grey solid (22.4g, 61%, MS: [M+H]+ = 570.6).

### Step 3) Preparation of Compound 8

Compound Sub 7(30 g, 52.7 mmol) and 9H-carbazole-d8(9.2 g, 52.7 mmol) were added to 240 ml of dimethylacetamide under a nitrogen atmosphere, and the mixture was stirred and refluxed. Then, potassium phosphate tribasic(33.5 g, 158 mmol) was added thereto, sufficiently stirred, and then reacted for 3 hours. The reaction mixture was cooled to room temperature, the organic layer was filtered to remove salt, and then the filtered organic layer was distilled. This was again added to and dissolved in 1142 mL of chloroform, washed twice with water, and the organic layer was separated. Anhydrous magnesium sulfate was added thereto, stirred, filtered, and the filtrate was distilled under reduced pressure. The concentrated compound was purified by a silica column using chloroform and ethyl acetate to prepare Compound 8 as a yellow solid (25.5 g, 67%, MS: [M+H]+ = 723.9).

### Preparation Example 9: Preparation of Compound 9

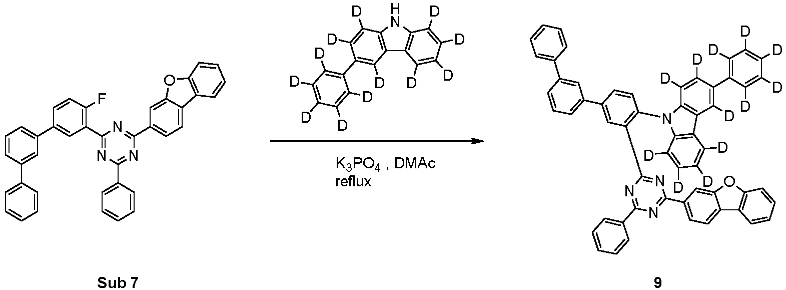

Compound 9 (34.1 g, 69%, MS: [M+H]+ = 806) was prepared in the same manner as in the preparation method of Compound 8, except that in Preparation Example 8, 3-(phenyl-d5)-9H-carbazole-1,2,4,5,6,7,8-d7 was used instead of 9H-carbazole-d8.

### Preparation Example 10: Preparation of Compound 10

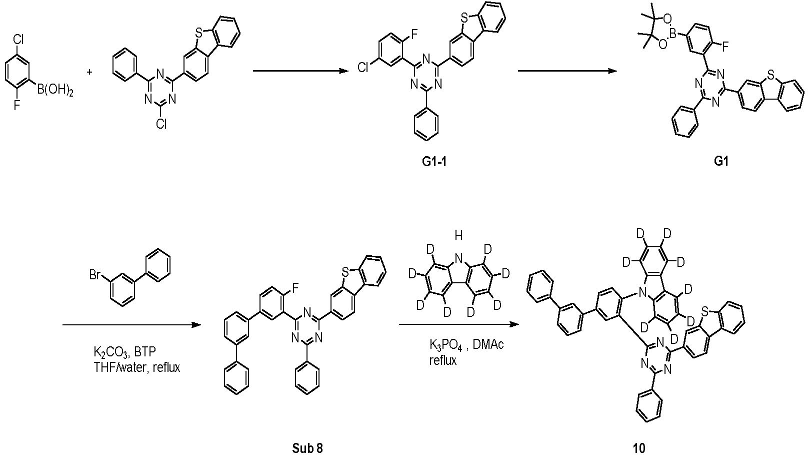

### Step 1) Preparation of Compound G1

Compound G1 (26.6 g, 52%, MS: [M+H]+ = 560.5) was prepared in the same manner as in the preparation method of Compound A1, except that in Preparation Example 1, 2-chloro-4-(dibenzo[b,d]thiophen-3-yl)-6-phenyl-1,3,5-triazine was used instead of 2-chloro-4,6-diphenyl-1,3,5-triazine.

### Step 2) Preparation of Compound Sub 8

Compound Sub 8 (26.4 g, 72%, MS: [M+H]+ = 586.7) was prepared in the same manner as in the preparation method of Compound Sub 7, except that in Preparation Example 8, Compound G1 was used instead of Compound F1.

### Step 3) Preparation of Compound 10

Compound Sub 8(25 g, 42.7 mmol) and 9H-carbazole-d8(7.5 g, 42.7 mmol) were added to 200 ml of dimethylacetamide under a nitrogen atmosphere, and the mixture was stirred and refluxed. Then, potassium phosphate tribasic(27.2 g, 128.1 mmol) was added thereto, sufficiently stirred, and then reacted for 2 hours. The reaction mixture was cooled to room temperature, the organic layer was filtered to remove salt, and then the filtered organic layer was distilled. This was again added to and dissolved in 949 mL of chloroform, washed twice with water, and the organic layer was separated. Anhydrous magnesium sulfate was added thereto, stirred, filtered, and the filtrate was distilled under reduced pressure. The concentrated compound was purified by a silica column using chloroform and ethyl acetate to prepare Compound 10 as a yellow solid (25.3 g, 80%, MS: [M+H]+ = 742).

### Preparation Example 11: Preparation of Compound 11

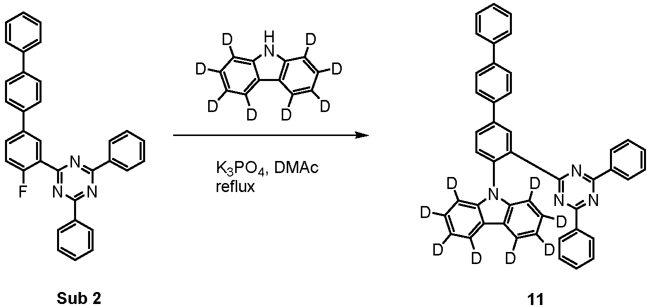

Compound 11(21.5 g, 70%, MS: [M+H]+ = 634) was prepared in the same manner as in the preparation method of Compound 2, except that in Preparation Example 2, 9H-carbazole-d8 was used instead of 9H-carbazole-d6.

### Preparation Example 12: Preparation of Compound 12

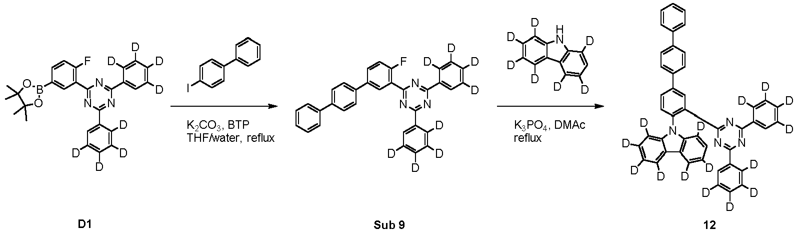

### Step 1) Preparation of Compound Sub 9

Compound D1(35 g, 75.9 mmol) and 4-iodo-1,1'-biphenyl(21.2 g, 75.9 mmol) were added to 350 ml of tetrahydrofuran under a nitrogen atmosphere, and the mixture was stirred and refluxed. Then, potassium carbonate(31.5 g, 227.6mmol) was dissolved in 31 ml of water, added thereto, sufficiently stirred, and then bis(tri-tert-butylphosphine)palladium(0.8 g, 1.5 mmol) was added. After the reaction for 5 hours, the reaction mixture was cooled to room temperature, the resulting solid was filtered. The solid was added to and dissolved in 1850 mL of chloroform, washed twice with water, and the organic layer was separated. Anhydrous magnesium sulfate was added thereto, stirred, filtered, and the filtrate was distilled under reduced pressure. The concentrated compound was purified by a silica column using chloroform and ethyl acetate to prepare Compound Sub 9 as a grey solid (27g, 73%, MS: [M+H]+= 488).

### Step 2) Preparation of Compound 12

Compound Sub 9(25 g, 51.3 mmol) and 9H-carbazole-1,2,3,4,5,6,8-d7(8.9 g, 51.3 mmol) were added to 200 ml of dimethylacetamide under a nitrogen atmosphere, and the mixture was stirred and refluxed. Then, potassium phosphate tribasic(32.6 g, 153.8 mmol) was added thereto, sufficiently stirred, and then reacted for 1 hour. The reaction mixture was cooled to room temperature, the organic layer was filtered to remove salt, and then the filtered organic layer was distilled. This was again added to and dissolved in 987 mL of chloroform, washed twice with water, and the organic layer was separated. Anhydrous magnesium sulfate was added thereto, stirred, filtered, and the filtrate was distilled under reduced pressure. The concentrated compound was purified by a silica column using chloroform and ethyl acetate to prepare Compound 12 as a yellow solid (17.4g, 53%, MS: [M+H]+= 642).

### Preparation Example 13: Preparation of Compound 13

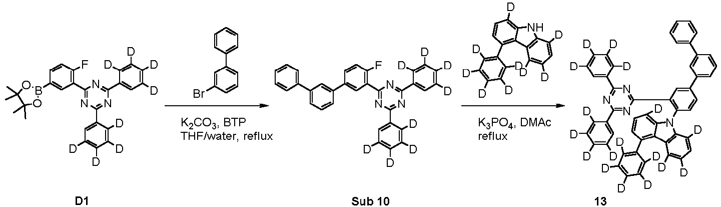

### Step 1) Preparation of Compound Sub 10

Compound D1(35 g, 75.9 mmol) and 3-bromo-1,1'-biphenyl(17.7 g, 75.9 mmol) were added to 350 ml of tetrahydrofuran under a nitrogen atmosphere, and the mixture was stirred and refluxed. Then, potassium carbonate(31.5 g, 227.6mmol) was dissolved in 31 ml of water, added thereto, sufficiently stirred, and then bis(tri-tert-butylphosphine)palladium(0.8 g, 1.5 mmol) was added. After the reaction for 4 hours, the reaction mixture was cooled to room temperature, the resulting solid was filtered. The solid was added to and dissolved in 1850 mL of chloroform, washed twice with water, and the organic layer was separated. Anhydrous magnesium sulfate was added thereto, stirred, filtered, and the filtrate was distilled under reduced pressure. The concentrated compound was purified by a silica column using chloroform and ethyl acetate to prepare Compound Sub 10 as a grey solid (29.2 g, 79%, MS: [M+H]+= 488.6).

### Step 2) Preparation of Compound 13

Compound Sub 10(25 g, 51.3 mmol) and 4-(phenyl-d5)-9H-carbazole-1,5,6,8-d4(12.9 g, 51.3 mmol) were added to 200 ml of dimethylacetamide under a nitrogen atmosphere, and the mixture was stirred and refluxed. Then, potassium phosphate tribasic(32.6 g, 153.8 mmol) was added thereto, sufficiently stirred, and then reacted for 2 hours. The reaction mixture was cooled to room temperature, the organic layer was filtered to remove salt, and then the filtered organic layer was distilled. This was again added to and dissolved in 1093 mL of chloroform, washed twice with water, and the organic layer was separated. Anhydrous magnesium sulfate was added thereto, stirred, filtered, and the filtrate was distilled under reduced pressure. The concentrated compound was purified by a silica column using chloroform and ethyl acetate to prepare Compound 13 as a yellow solid (23.7g, 65%, MS: [M+H]+ = 720).

### Preparation Example 14: Preparation of Compound 14

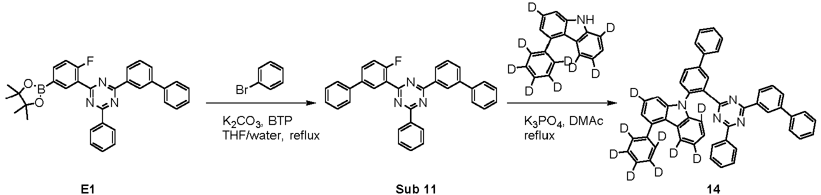

### Step 1) Preparation of Compound Sub 11

Compound E1(35 g, 66.1 mmol) and bromobenzene(10.4 g, 66.1 mmol) were added to 350 ml of tetrahydrofuran under a nitrogen atmosphere, and the mixture was stirred and refluxed. Then, potassium carbonate(27.4 g, 198.3mmol) was dissolved in 27 ml of water, added thereto, sufficiently stirred, and then bis(tri-tert-butylphosphine)palladium(0.7 g, 1.3 mmol) was added. After the reaction for 3 hours, the reaction mixture was cooled to room temperature, the resulting solid was filtered. The solid was added to and dissolved in 1585 mL of chloroform, washed twice with water, and the organic layer was separated. Anhydrous magnesium sulfate was added thereto, stirred, filtered, and the filtrate was distilled under reduced pressure. The concentrated compound was purified by a silica column using chloroform and ethyl acetate to prepare Compound Sub 11 as a grey solid (25.4g, 80%, MS: [M+H]+ = 480.6).

### Step 2) Preparation of Compound 14

Compound Sub 11(25 g, 52.1 mmol) and 4-(phenyl-d5)-9H-carbazole-2,5,6,8-d4(13.2 g, 52.1 mmol) were added to 200 ml of dimethylacetamide under a nitrogen atmosphere, and the mixture was stirred and refluxed. Then, potassium phosphate tribasic (33.2 g, 156.4 mmol) was added thereto, sufficiently stirred, and then reacted for 3 hours. The reaction mixture was cooled to room temperature, the organic layer was filtered to remove salt, and then the filtered organic layer was distilled. This was again added to and dissolved in 1099 mL of chloroform, washed twice with water, and the organic layer was separated. Anhydrous magnesium sulfate was added thereto, stirred, filtered, and the filtrate was distilled under reduced pressure. The concentrated compound was purified by a silica column using chloroform and ethyl acetate to prepare Compound 14 as a yellow solid (23.1g, 63%, MS: [M+H]+ = 712).

### [EXAMPLE]

### Example 1: Manufacture of organic light emitting device

A glass substrate on which ITO (indium tin oxide) was coated as a thin film to a thickness of 1400 Å was put into distilled water in which a detergent was dissolved, and ultrasonically cleaned. A product manufactured by Fischer Co. was used as the detergent, and as the distilled water, distilled water filtered twice using a filter manufactured by Millipore Co. was used. After the ITO was cleaned for 30 minutes, ultrasonic cleaning was repeated twice using distilled water for 10 minutes. After the cleaning with distilled water was completed, the substrate was ultrasonically cleaned with solvents of isopropyl alcohol, acetone, and methanol, dried, and then transferred to a plasma cleaner. In addition, the substrate was cleaned for 5 minutes using oxygen plasma and then transferred to a vacuum depositor.

On the ITO transparent electrode thus prepared, the following compound HT-A and the following compound PD were thermally vacuum-deposited at a weight ratio of 95:5 to a thickness of 100 Å, and then only the following compound HT-A was deposited to a thickness of 1150 Å to form a hole transport layer. The following compound HT-B was thermally vacuum-deposited to a thickness of 450 Å on the hole transport layer to form an electron blocking layer. The previously prepared compound 1 and the following compound GD were vacuum-deposited at a weight ratio of 85:15 to a thickness of 400 Å on the electron blocking layer to form a light emitting layer. The following compound ET-A was vacuum-deposited to a thickness of 50 Å on the light emitting layer to form a hole blocking layer. The following compound ET-B and the following compound Liq were thermally vacuum-deposited at a weight ratio of 2:1 to a thickness of 250 Å on the hole blocking layer, and then Lif and magnesium were vacuum-deposited at a weight ratio of 1:1 to a thickness of 30 Å to form an electron injection and transport layer. Magnesium and silver were deposited at a weight ratio of 1: 4 to a thickness of 160 Å on the electron injection layer to form a cathode, thereby completing the manufacture of an organic light emitting device.

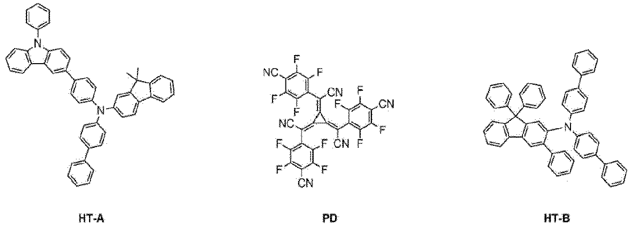

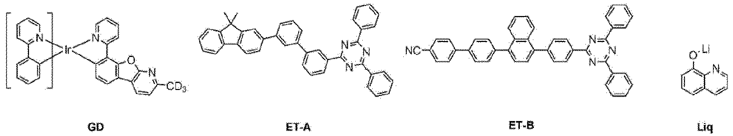

In the above-mentioned processes, the vapor deposition rate of the organic material was maintained at 0.4 ~ 0.7 Å/sec, the deposition rate of magnesium and silver was maintained at 2 Å/sec, and the degree of vacuum during the deposition was maintained at 2*10⁻⁷ ~ 5*10⁻⁶ torr, thereby manufacturing an organic light emitting device.

### Examples 2 to 12 and Comparative Examples 1 to 9

The organic light emitting devices of Examples 2 to 12 and Comparative Examples 1 to 9 were manufactured in the same manner as in Example 1, except that the host material was changed as shown in Table 1 below. In Table 1, each of Compounds GH-A, GH-B, GH-C, GH-D, GH-E, GH-F, GH-G, GH-H and GH-I is as follows. D₁₄ in the following compound GH-C means that 14 hydrogens in the compound given in parentheses are replaced with deuterium.

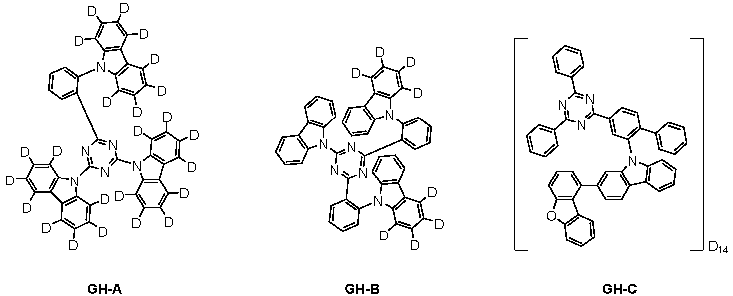

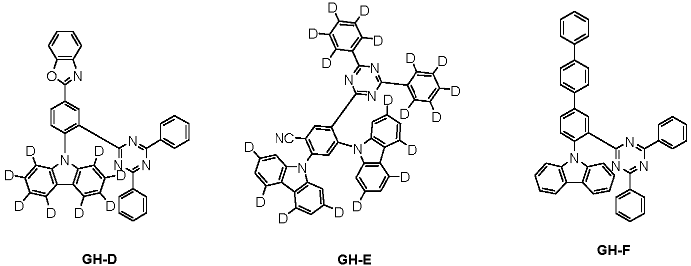

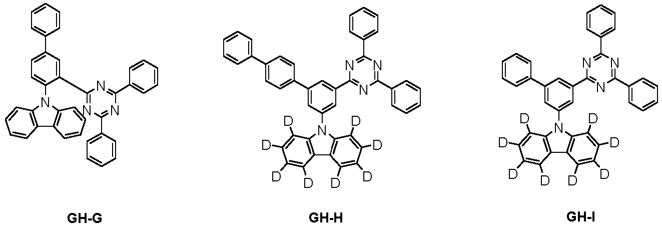

### [Experimental Example]

The voltage, efficiency, and lifetime (T95) were measured by applying a current to the organic light emitting devices manufactured in Examples and Comparative Examples, and the results are shown in Table 1 below. At this time, the voltage and efficiency were measured by applying a current density of 10 mA/cm². Further, T95 shown in Table 1 means the time required for the luminance to be reduced to 95% of the initial luminance at a current density of 20 mA/cm².

**[Table 1]**

| | Light emitting layer (host) | Voltage (V) | Efficiency (cd/A) | Lifetime (T95, hr) |
|---|---|---|---|---|
| Example 1 | Compound 1 | 4.75 | 39.7 | 92 |
| Example 2 | Compound 2 | 4.91 | 35.1 | 89 |
| Example 3 | Compound 3 | 4.12 | 33.0 | 83 |
| Example 4 | Compound 4 | 4.44 | 32.9 | 88 |
| Example 5 | Compound 5 | 4.62 | 36.5 | 72 |
| Example 6 | Compound 6 | 4.88 | 36.1 | 66 |
| Example 7 | Compound 7 | 4.83 | 37.1 | 74 |
| Example 8 | Compound 8 | 4.47 | 33.3 | 83 |
| Example 9 | Compound 9 | 5.00 | 38.4 | 79 |
| Example 10 | Compound 10 | 4.95 | 38.1 | 77 |
| Example 11 | Compound 11 | 4.91 | 37.3 | 98 |
| Example 12 | Compound 12 | 4.91 | 39.6 | 108 |
| Example 13 | Compound 13 | 4.10 | 56.2 | 138 |
| Example 14 | Compound 14 | 4.02 | 59.6 | 156 |
| Comparative Example 1 | GH-A | 5.95 | 22.1 | 16 |
| Comparative Example 2 | GH-B | 6.02 | 11.2 | 10 |
| Comparative Example 3 | GH-C | 5.44 | 28.2 | 13 |
| Comparative Example 4 | GH-D | 5.21 | 23.1 | 49 |
| Comparative Example 5 | GH-E | 5.72 | 20.0 | 52 |
| Comparative Example 6 | GH-F | 5.90 | 24.1 | 46 |
| Comparative Example 7 | GH-G | 5.72 | 26.0 | 41 |
| Comparative Example 8 | GH-H | 5.52 | 19.6 | 48 |
| Comparative Example 9 | GH-I | 5.32 | 20.8 | 39 |

The compound represented by Chemical Formula 1 has a structure in which a polycyclic nitrogen-containing heterocyclic substituent that functions as an intramolecular electron donor and a monocyclic nitrogen-containing heterocycle that functions as an electron acceptor are bonded to each other at the ortho position, which makes it possible to easily achieve intra charge transfer.

In addition, compounds in which a benzene ring that functions as an electron donor, and a polycyclic nitrogen-containing hetero ring are bonded to each other at the para position within the molecule have high molecular stability and are favorable for both hole and electron transport.

Therefore, as shown in Table 1, it can be confirmed that low voltage, high efficiency, and long lifetime characteristics appear when the compound of Chemical Formula 1 is used as a host for an organic light emitting device.

### [Description of Symbols]

| | | | |
|---|---|---|---|
| 1: | substrate | 2: | anode |
| 3: | light emitting layer | 4: | cathode |
| 5: | hole injection layer | 6: | hole transport layer |
| 7: | electron blocking layer | 8: | hole blocking layer |
| 9: | electron transport layer | 10: | electron injection layer |

## Claims

1. A compound represented by the following Chemical Formula 1: 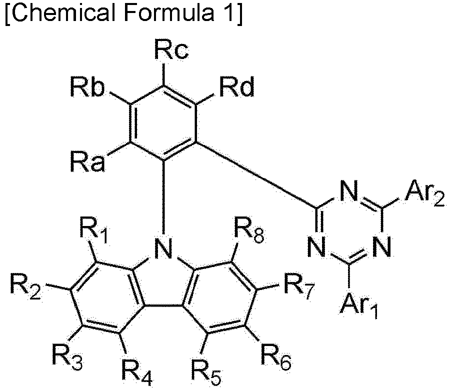 in Chemical Formula 1,
Ar₁ and Ar₂ are each independently a substituted or unsubstituted C₆₋₆₀ aryl; or a substituted or unsubstituted C₂₋₆₀ heteroaryl containing at least one selected from the group consisting of O and S,
R₁ to R₈ are each independently hydrogen; deuterium; or a substituted or unsubstituted C₆₋₆₀ aryl, with the proviso that at least one of R₁ to R₈ is deuterium, and
Ra to Rd are each independently hydrogen; deuterium; or a C₆₋₆₀ aryl unsubstituted or substituted with deuterium.

2. The compound as claimed in claim 1, wherein:
Ar₁ and Ar₂ are each independently phenyl, biphenylyl, dibenzofuranyl, or dibenzothiophenyl,
wherein the Ar₁ and Ar₂ are each independently unsubstituted or substituted with at least one deuterium.

3. The compound as claimed in claim 1, wherein:
at least one of Ar₁ and Ar₂ is phenyl unsubstituted or substituted with at least one deuterium.

4. The compound as claimed in claim 1, wherein:
R₁ to R₈ are each independently hydrogen, deuterium, or phenyl substituted with five deuterium, with the proviso that least one of R₁ to R₈ is deuterium.

5. The compound as claimed in claim 1, wherein:
four or more of R₁ to R₈ are deuterium.

6. The compound as claimed in claim 1, wherein:
Ra to Rd are each independently hydrogen, deuterium, phenyl, biphenylyl, terphenylyl, naphthyl, anthracenyl, phenanthrenyl, or triphenylenyl,
wherein the phenyl, biphenylyl, terphenylyl, naphthyl, anthracenyl, phenanthrenyl, or triphenylenyl are each independently unsubstituted or substituted with at least one deuterium.

7. The compound as claimed in claim 1, wherein:
Ra to Rd are each independently hydrogen, phenyl, biphenylyl, terphenylyl, naphthyl, anthracenyl, phenanthrenyl, or triphenylenyl,
wherein the phenyl, biphenylyl, terphenylyl, naphthyl, anthracenyl, phenanthrenyl, or triphenylenyl are each independently unsubstituted or substituted with at least one deuterium.

8. The compound as claimed in claim 1, wherein:
any one of Ra to Rd is phenyl, biphenylyl, terphenylyl, naphthyl, anthracenyl, phenanthrenyl, or triphenylenyl, wherein the phenyl, biphenylyl, terphenylyl, naphthyl, anthracenyl, phenanthrenyl, and triphenylenyl are each independently unsubstituted or substituted with at least one deuterium, and
the rest is hydrogen.

9. The compound as claimed in claim 1, wherein:
the compound represented by Chemical Formula 1 is any one selected from the group consisting of compounds of the following:

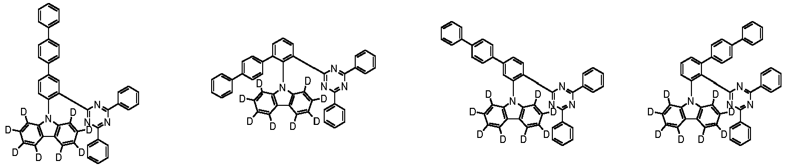

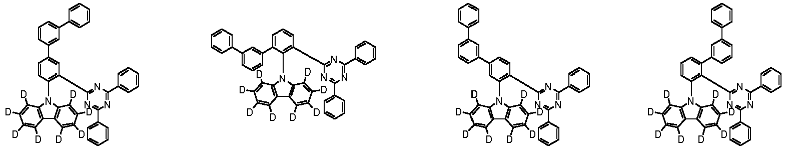

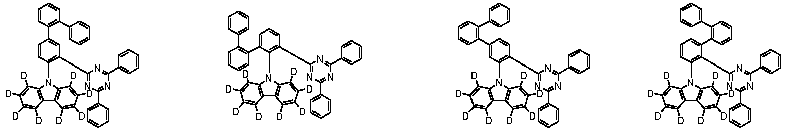

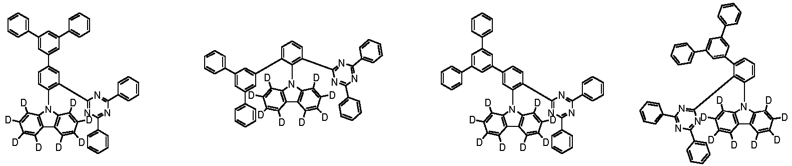

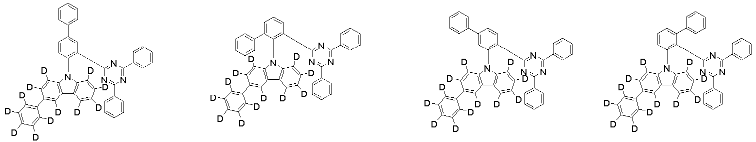

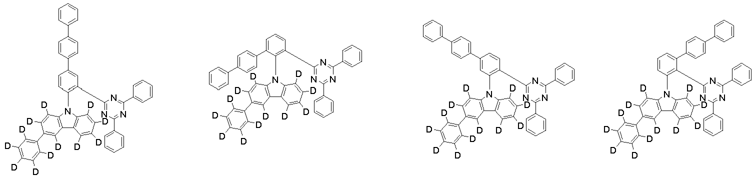

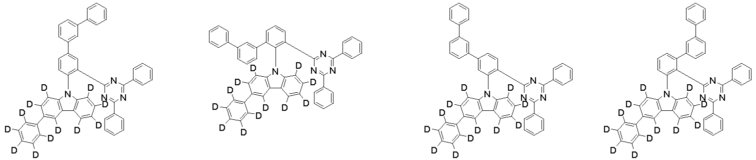

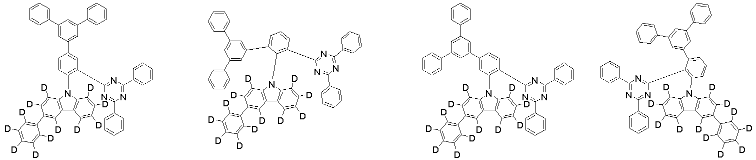

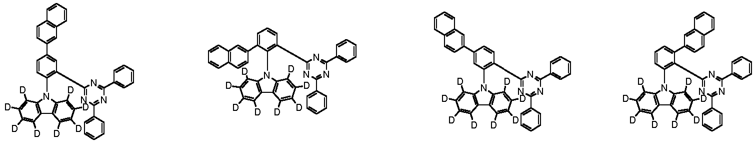

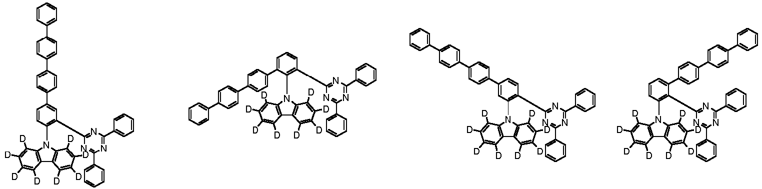

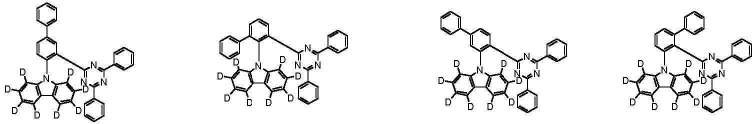

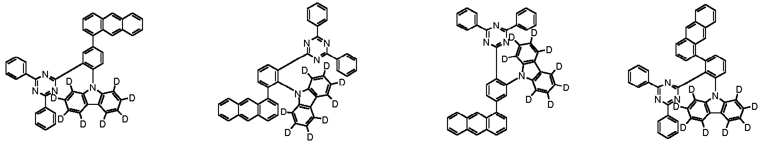

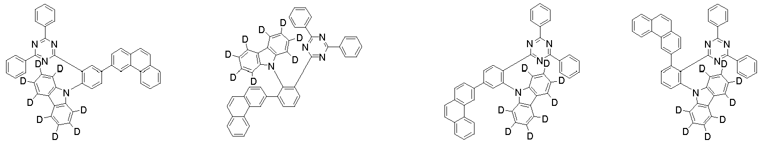

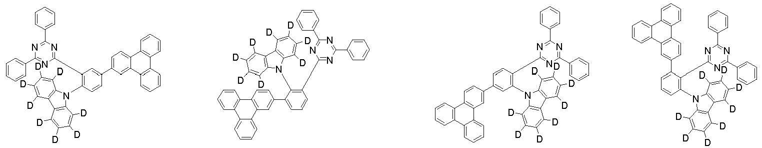

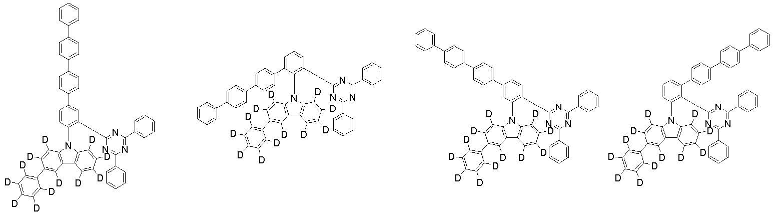

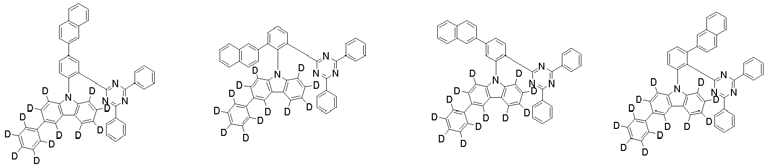

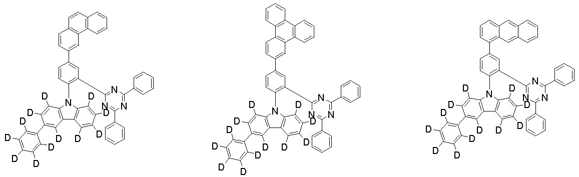

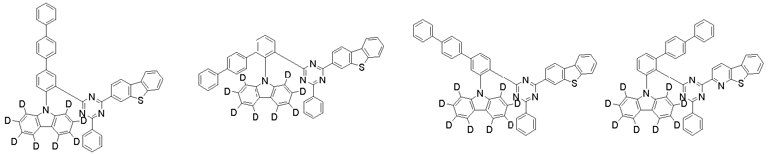

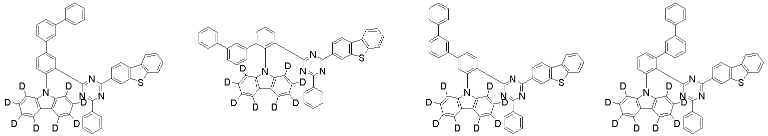

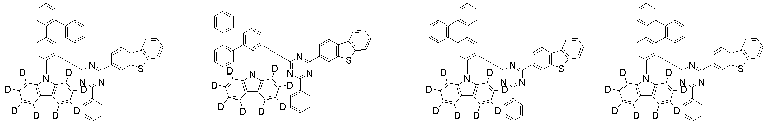

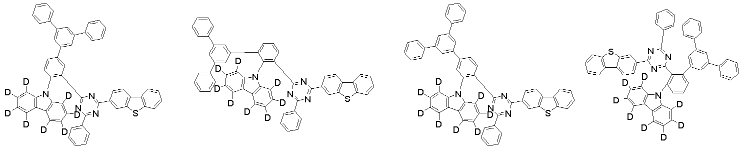

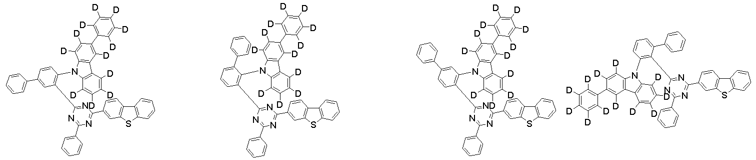

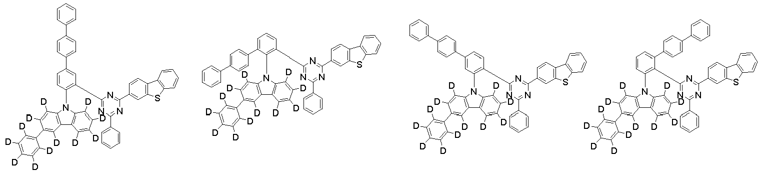

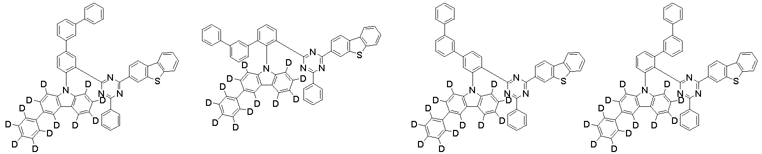

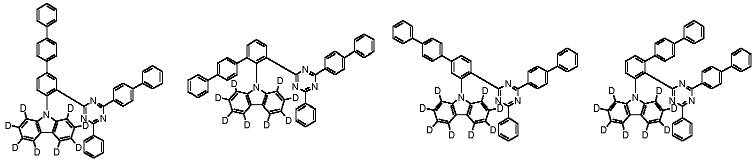

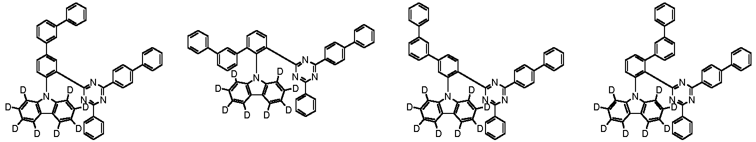

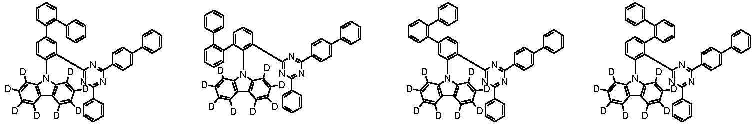

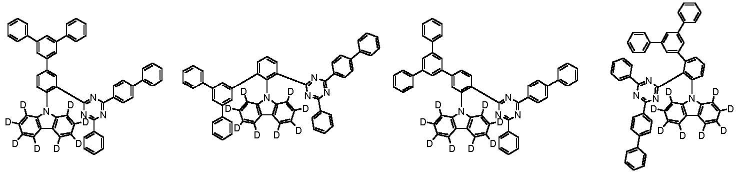

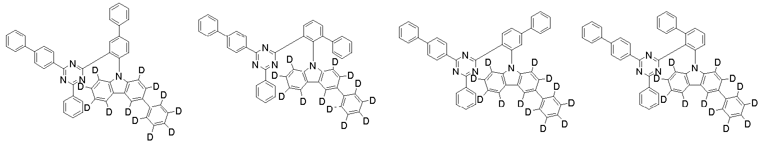

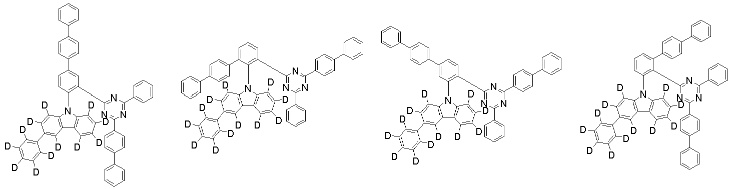

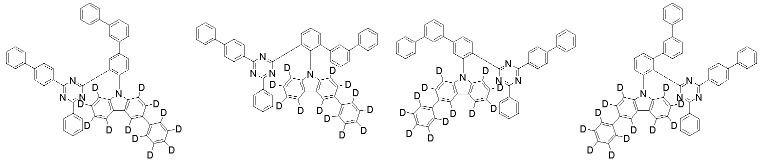

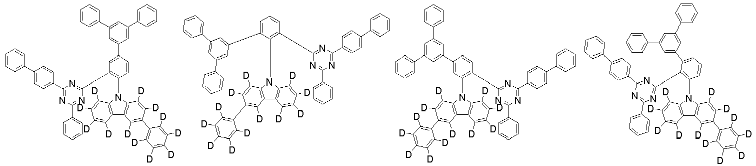

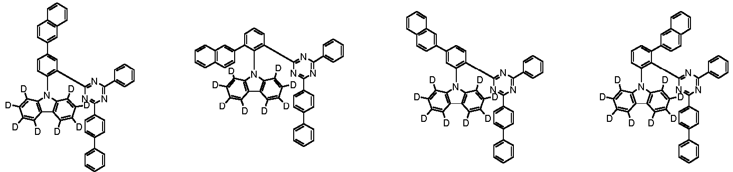

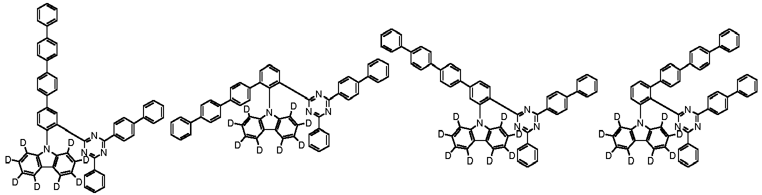

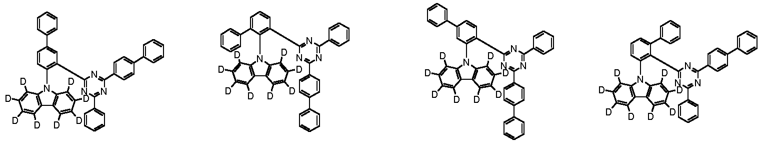

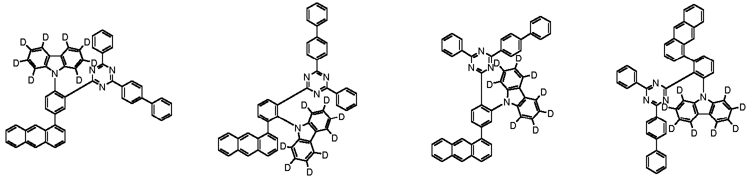

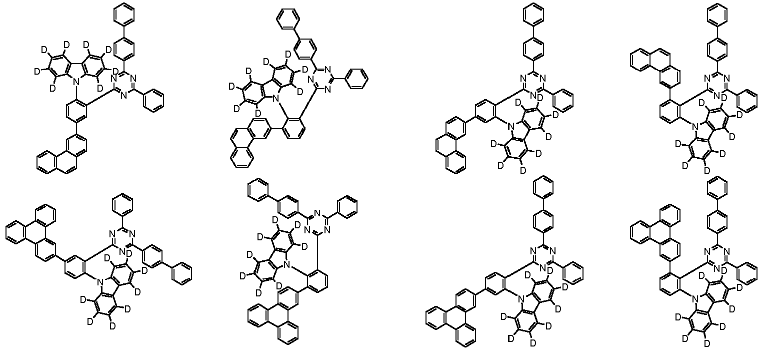

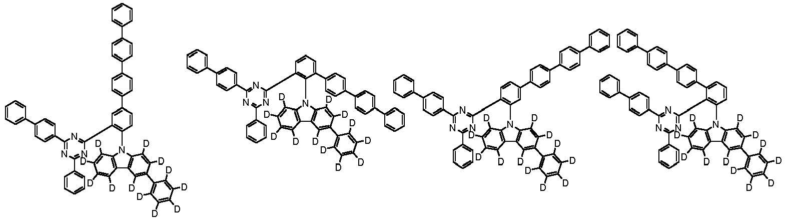

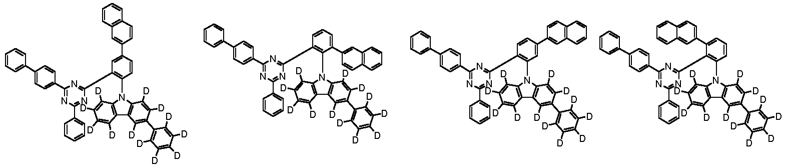

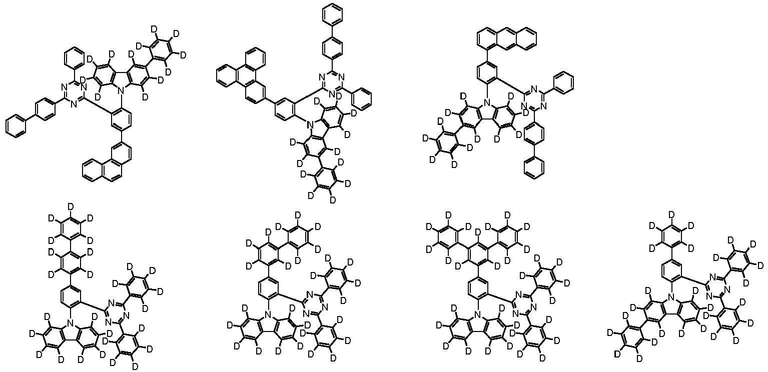

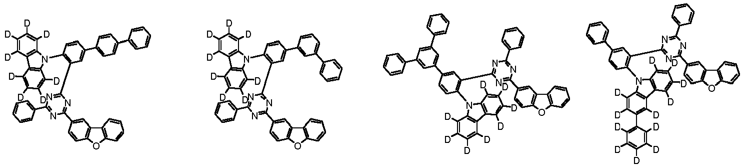

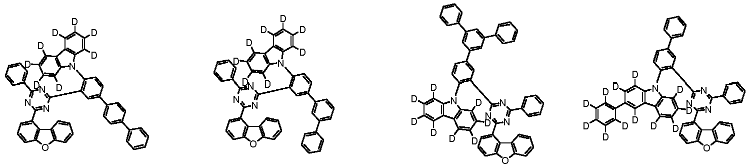

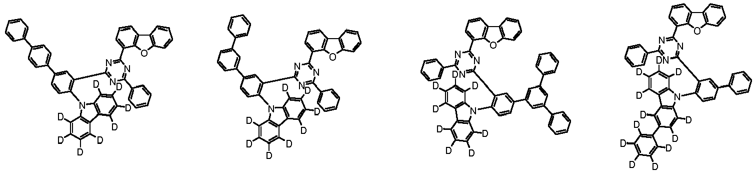

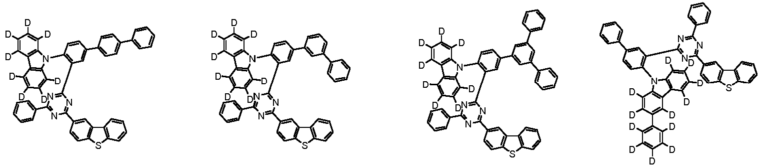

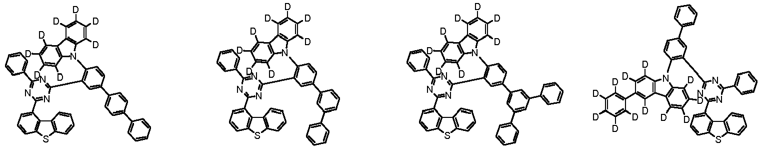

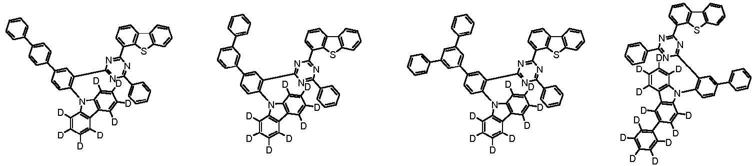

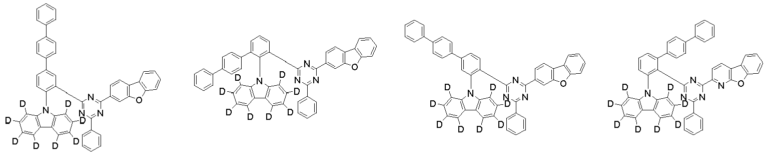

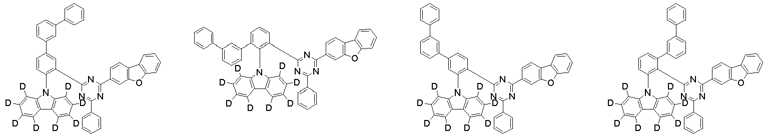

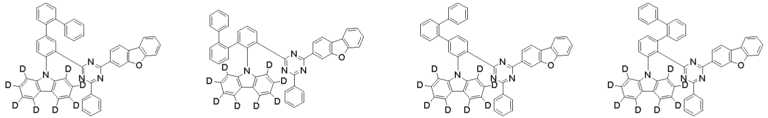

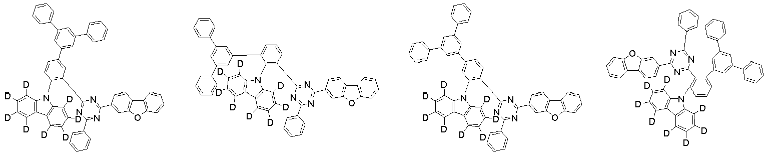

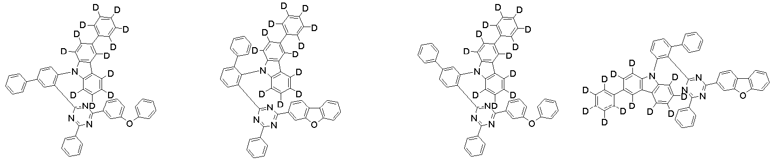

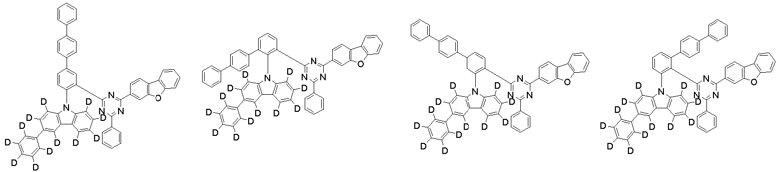

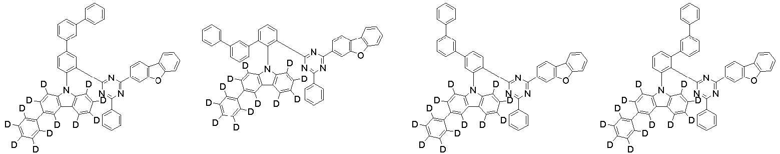

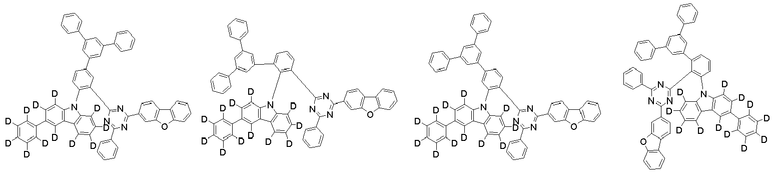

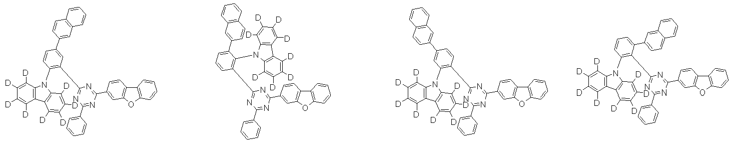

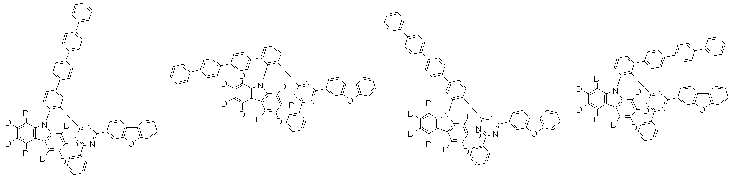

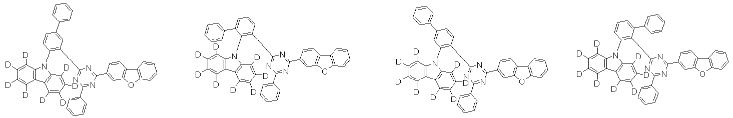

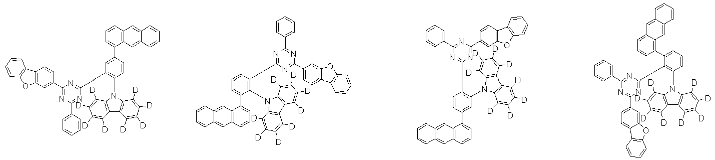

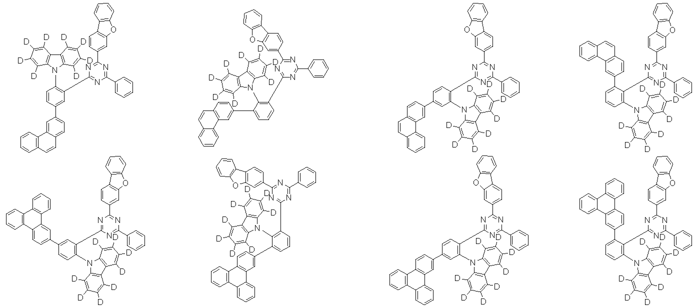

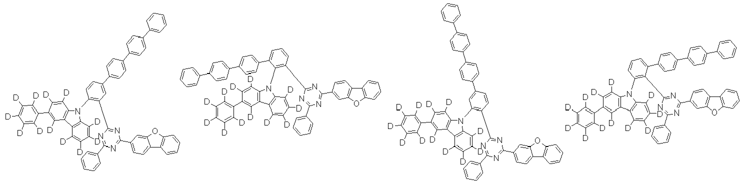

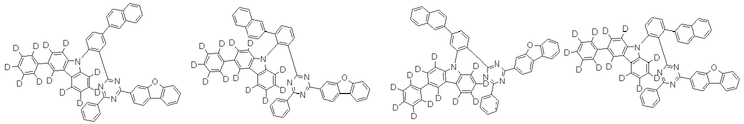

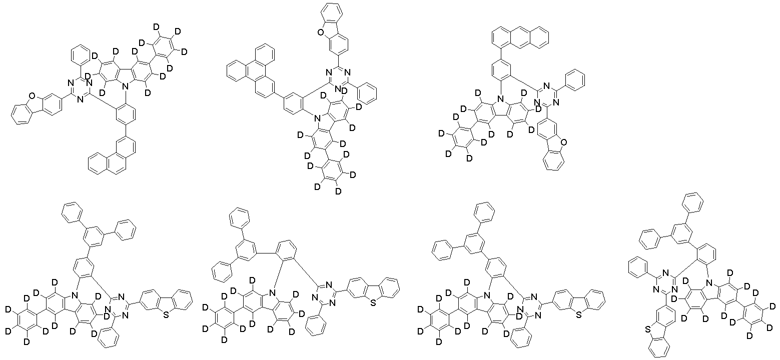

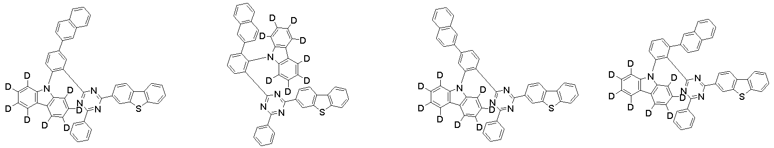

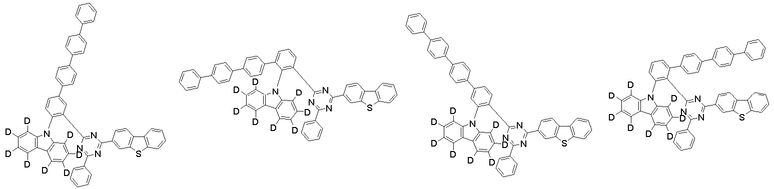

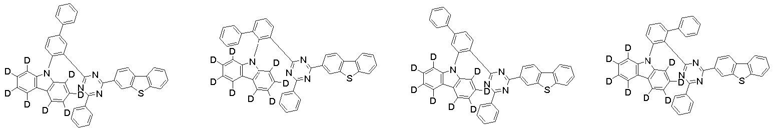

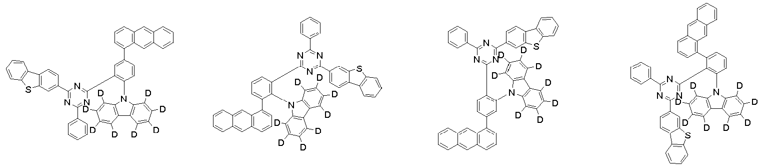

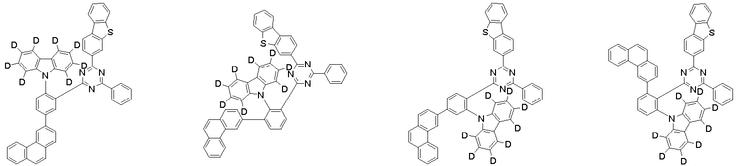

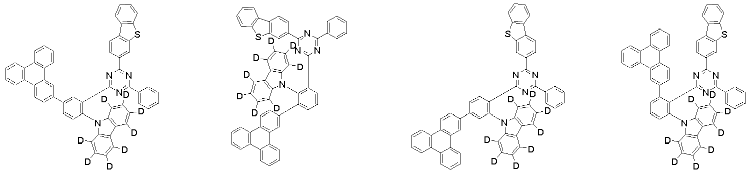

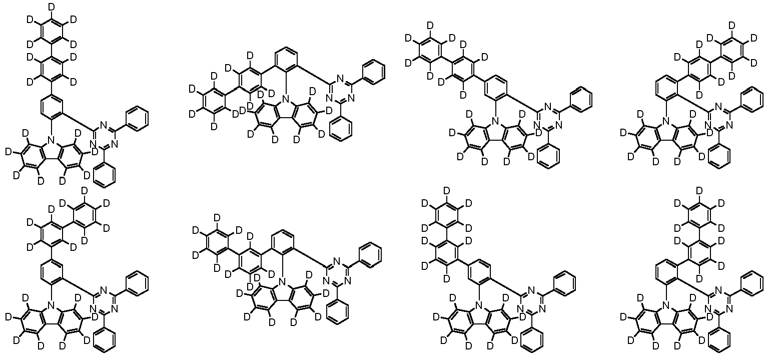

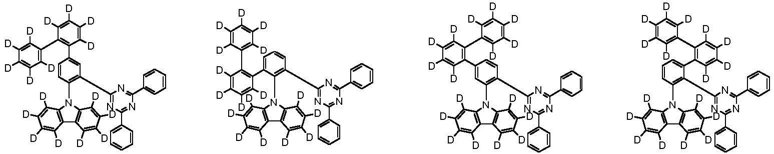

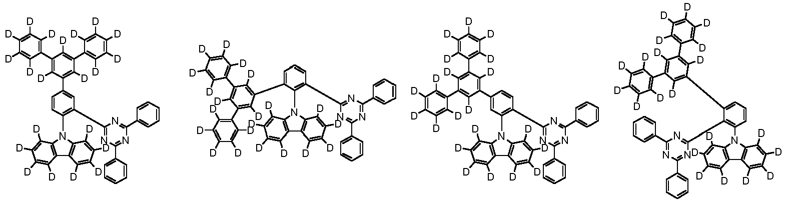

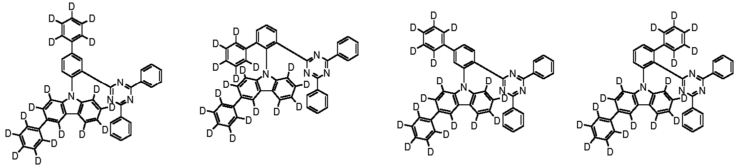

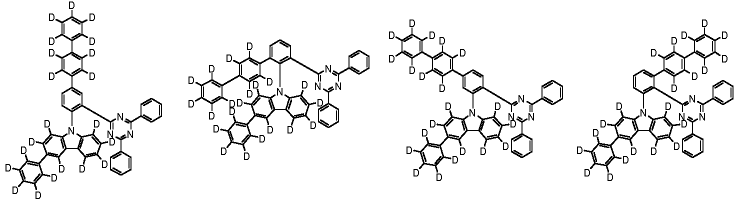

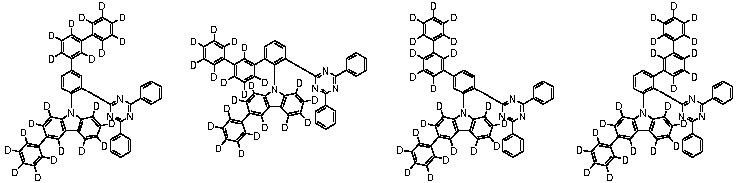

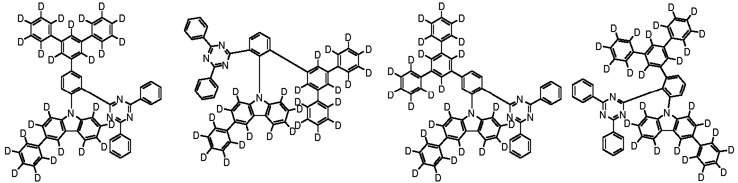

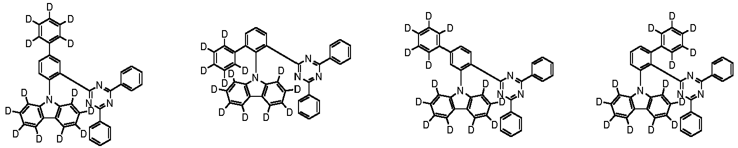

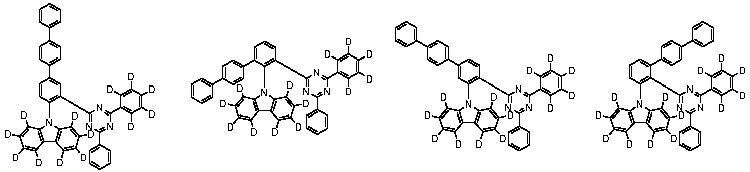

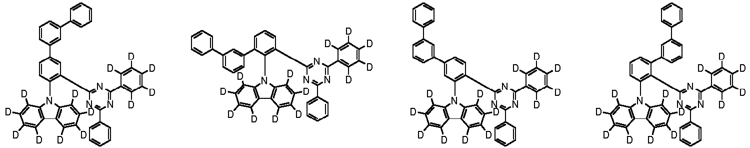

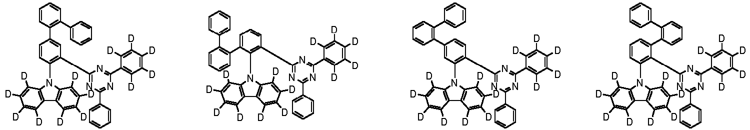

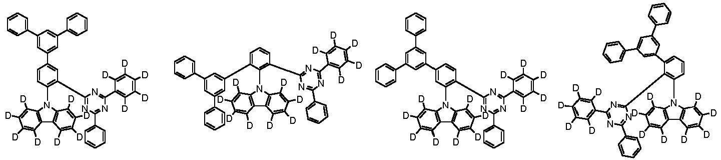

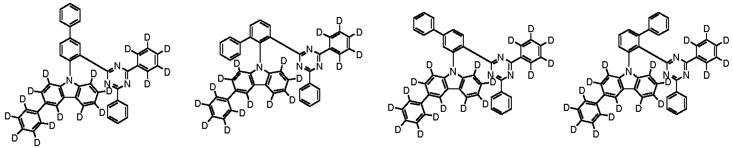

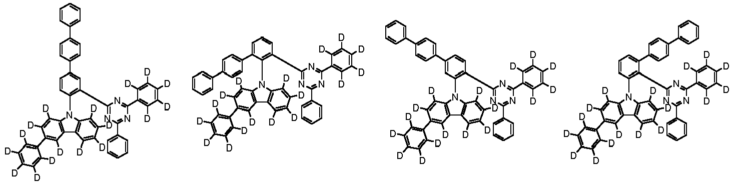

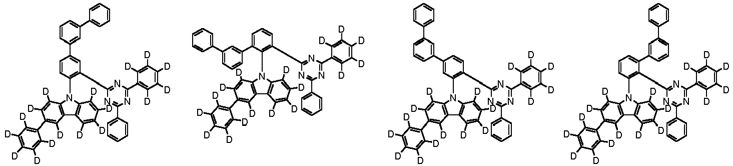

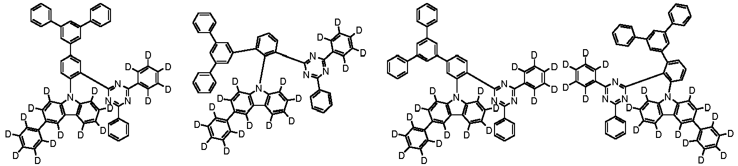

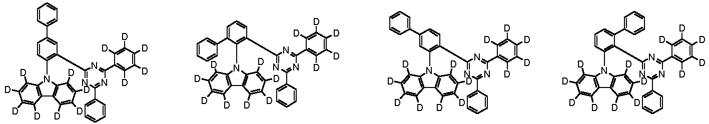

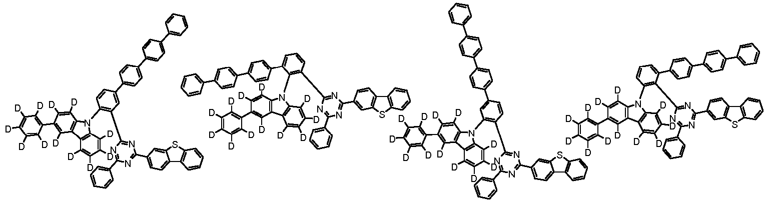

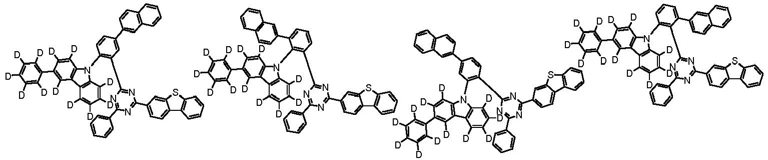

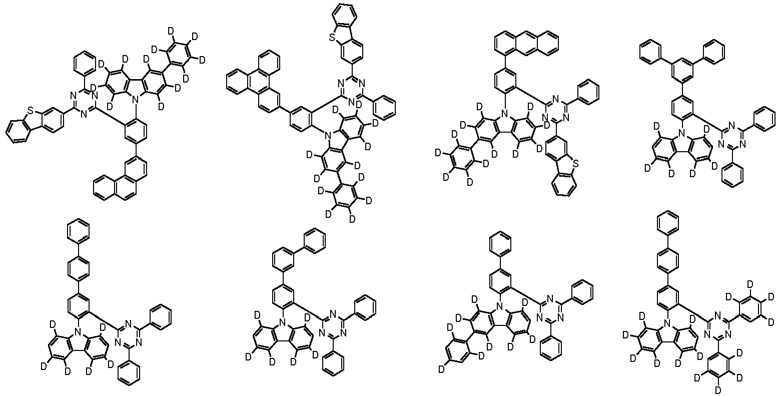

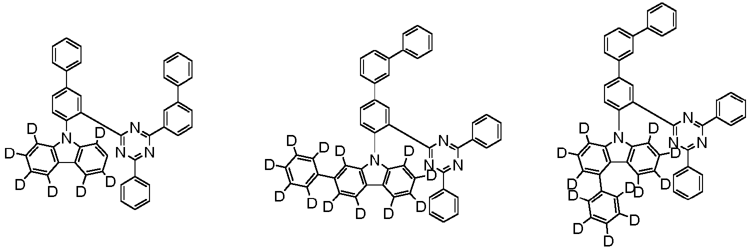

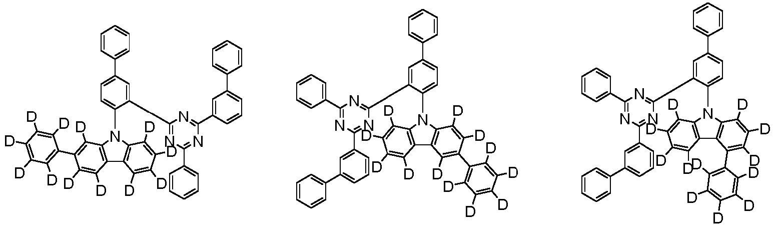

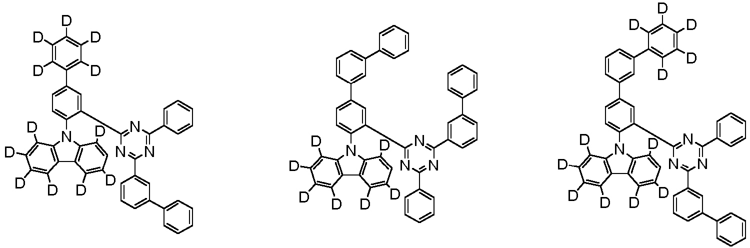

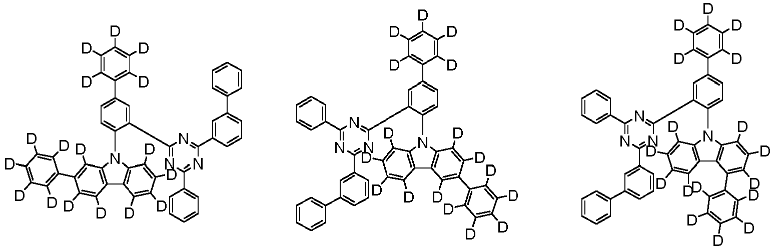

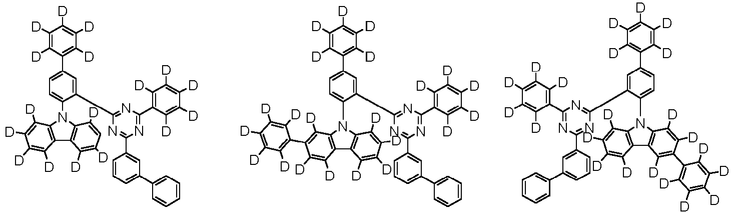

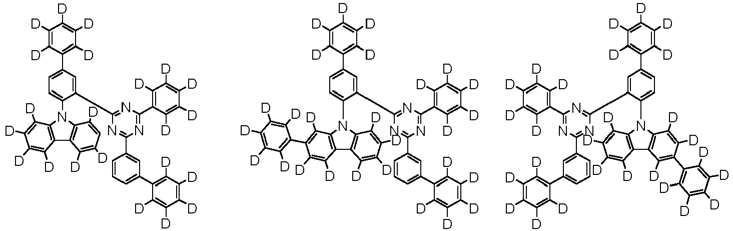

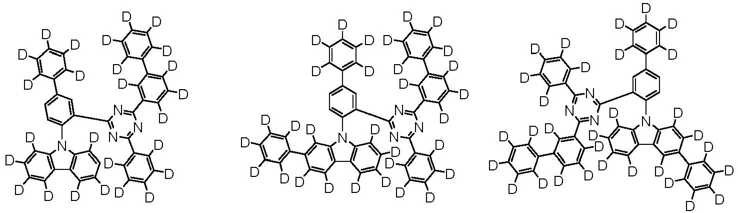

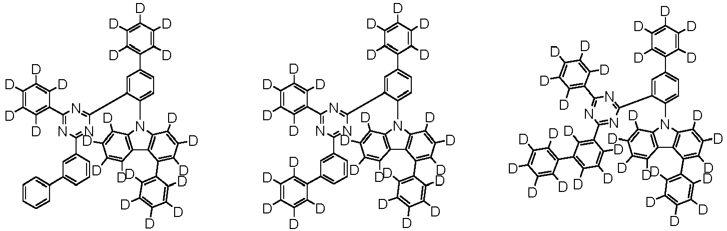

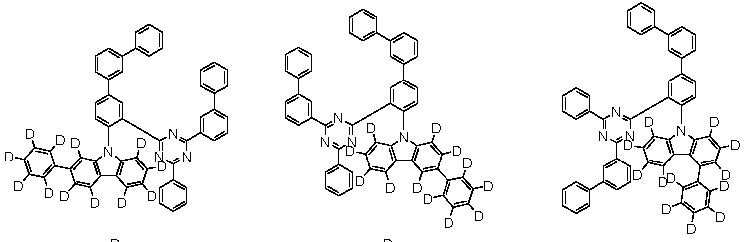

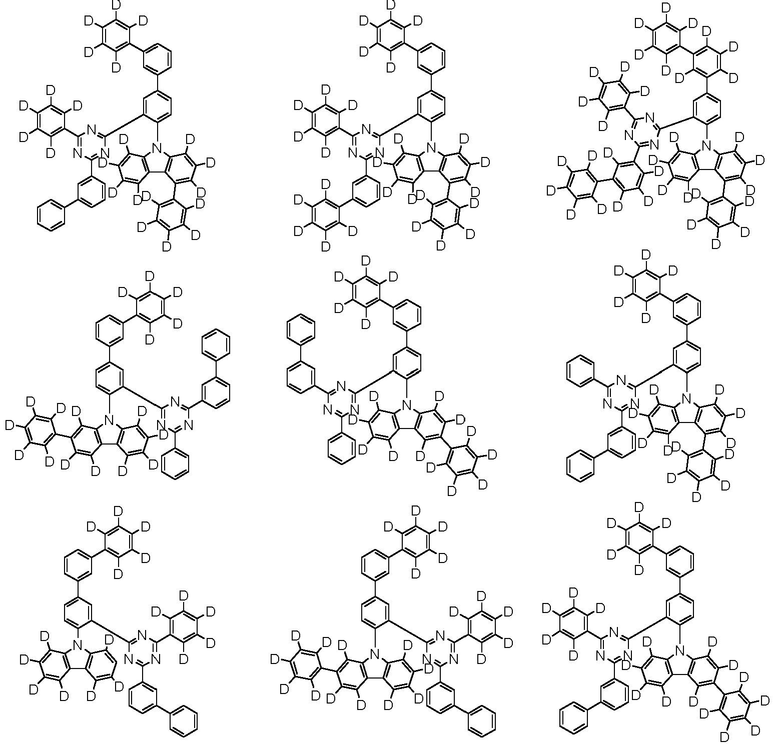

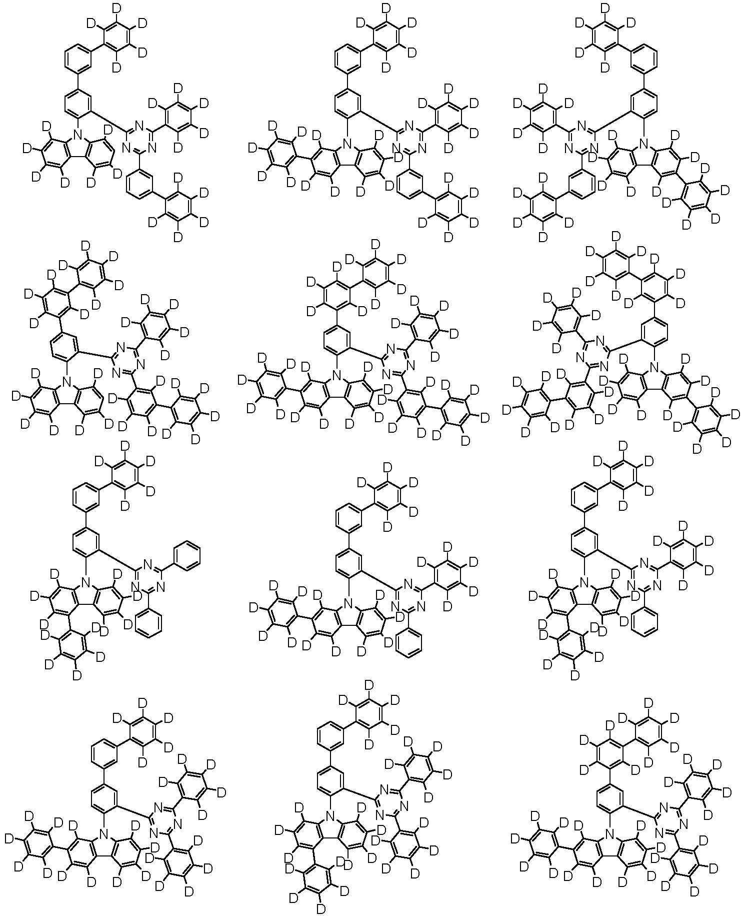

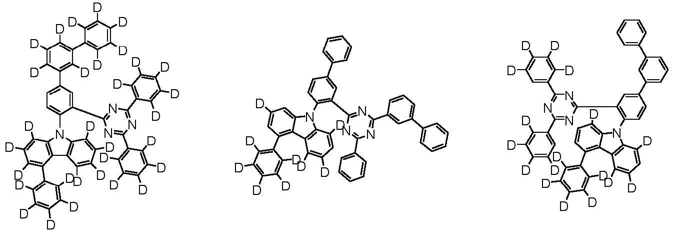

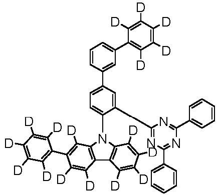

10. An organic light emitting device comprising: a first electrode; a second electrode that is provided opposite to the first electrode; and one or more organic material layers that are provided between the first electrode and the second electrode, wherein at least one layer of the organic material layers comprises the compound as claimed in any one of claims 1 to 9.

11. An organic light emitting device as claimed in claim 10, wherein:
the organic material layer is a light emitting layer.
